(19) Europäisches Patentamt
European Patent Office
Office européen des brevets

(11) EP 2 640 832 B1

(12) EUROPEAN PATENT SPECIFICATION

(45) Date of publication and mention of the grant of the patent:
**20.12.2017 Bulletin 2017/51**

(21) Application number: **11841118.0**

(22) Date of filing: **15.11.2011**

(51) Int Cl.:
*C12N 7/00* (2006.01) *C12N 15/33* (2006.01)
*C12N 15/41* (2006.01) *C07K 14/085* (2006.01)
*C07K 16/10* (2006.01) *A61K 39/125* (2006.01)

(86) International application number:
**PCT/IB2011/055103**

(87) International publication number:
**WO 2012/066481 (24.05.2012 Gazette 2012/21)**

(54) **NEW ETHIOLOGICAL AGENT**

NEUES ÄTIOLOGISCHES MITTEL

NOUVEL AGENT ÉTIOLOGIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.11.2010 NO 20101604**

(43) Date of publication of application:
**25.09.2013 Bulletin 2013/39**

(73) Proprietor: **Pharmaq AS**
**7863 Overhalla (NO)**

(72) Inventors:
- **NILSEN, Pål**
**1151 Oslo (NO)**
- **FRØYSTAD-SAUGEN, Marianne**
**N-1920 Sørumsand (NO)**
- **LINDMO, Karine**
**N-0461 Oslo (NO)**
- **MIKALSEN, Aase Beathe**
**N-1182 Oslo (NO)**
- **EVENSEN, Øystein**
**N-0198 Oslo (NO)**
- **RODE, Marit**
**N-1170 Oslo (NO)**

(74) Representative: **Mannion, Sally Kim**
**Zoetis UK Limited**
**Walton Oaks**
**Dorking Road**
**Tadworth**
**Surrey KT20 7NS (GB)**

(56) References cited:
**WO-A1-2005/121325 CA-A1- 2 634 144**

- **DATABASE EMBL [Online] 15 September 2006 (2006-09-15), "PMAH-aab89g08.g1 Lamprey_EST_Olfactory Petromyzon marinus cDNA 5', mRNA sequence.", XP002730087, retrieved from EBI accession no. EM_EST:EE740799 Database accession no. EE740799**
- **MIKALSEN A B ET AL: "Characterization of a Novel Calicivirus Causing Systemic Infection in Atlantic Salmon (Salmo salar L.): Proposal for a New Genus of Caliciviridae", PLOS ONE, vol. 9, no. 9, 9 September 2014 (2014-09-09), page e107132, XP055141623, ISSN: 1932-6203, DOI: 10.1371/journal.pone.0107132**
- **DATABASE GENBANK 25 October 2002 'Salmo salar strain McConnell clone ssalrgb502150 EST', XP003033302 Database accession no. CA061334**
- **L'HOMME ET AL.: 'Genomic characterization of swine caliciviruses representing a new genus of Caliciviridae' VIRUS GENES vol. 39, no. 1, 2009, pages 66 - 75, XP019690394**
- **DATABASE GENBANK 17 December 1997 'RNA-dependent RNA polymerase, partial [Norovirus isolates)', XP003033303 Database accession no. BAA24151**
- **DATABASE GENBANK 10 December 2008 'Major capsid protein (Norovirus strain Hu/GII.4/CHDC2094/1974/US gene VP1)', XP003033304 Database accession no. ACT76142**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

(Cont. next page)

- DATABASE GENBANK 13 July 2005 'Polyprotein (sapovirus strain Hu/Angelholm/SW278/2004/SE)', XP003033305 Database accession no. ABA00798
- ESSBAUER ET AL.: 'Viruses of lower vertebrates' J. VET. MED. B vol. 48, no. 6, 2001, pages 403 - 475, XP055120384

SEQUENCE LISTING

<110> Pharmaq AS

<120> New ethiological agent

<130> 46446pc01

<150> NO20101604
<151> 2010-11-15

<160> 19

<170> PatentIn version 3.5

<210> 1
<211> 4761
<212> DNA
<213> Salmonid calicivirus

<400> 1

gtacaccacc ctggttgagg aactgcgcgc ggccgcgcca gttcagaagg ctgtcgctga 60
ggaggacgcc ccgatccaga acgaggcgta cgcctgcgcg gctgagacga acatgagtgt 120
tgcccaggtt ggcgctggcg aggacccgtt gaaggttttg ctgtcgttcg agagacacaa 180
cgttccgggg ctgaacatgc tgacctcctt ctctagtgtg gtcaagcaga ttcagatcca 240
gggcaagact cggacccatc ccaaatctct ggtgtgctcc atcaacgacg ctaagttggt 300
gctcaagatc ccgccatgca gcgtcaagtt tgcggatgcc gtcgtgaact tctggagcta 360
cgagggcaag ttgcggtgtg ccctagtcaa ggagggtgtc aaagagagtg ccgggttggg 420
tttctccacc tcacccagcg tggacccgtt tgccttcgac caagtggtcg aagatgagta 480
cgagctgccc aagtctatca gtggggcgct gctgaatgtg atgaccaccg tgctcgggat 540
ggtggcgatg gcgatggaag ccaaagtcac tttccacaca ccgaccgaag aggatgagaa 600
aggaccagct cgacgggtga ctcgatctta ccgacccgta cgggacagtg acttccagcc 660
agctggtcgc ctgtgggcgg aagatcggcg cgaaatgggc tacaccgaac ggattacgtt 720
cgattccaat aactggatcg tcccctgctt cgacgcggct gggtgcccaa aaggttgggc 780
gatctctacg cccaagggcc tgctggcgaa ccggcatgtg atcgctggga cgactaggct 840
tggccaccaa aaagttggcg agtccgtggt cctcaactac ggcgacactg acctttgcct 900
gatcaagaac accaagaacc cataccagct gtgtaaggcg cctttctccc gacccattgt 960
tggtgagatc ctttaccagc tgaagccatc tgggatgcag cgggctcgtg tccacagttt 1020
cgtccgggtg gccaacccaa gtggcaagct cgttgagatg tgcctggtcg aaggggggcgt 1080
cagtgaccct ggtgattgtg gcctgccatg ggtccgccgc gtgggccgaa gtgtggagtt 1140
ggtcggcctg gcagccggca ctcgaggggc gaggctgatg gtcgttccac tgggggttga 1200
cccagctgag acccagtggc atgccagaac ccacaactac acctttctgc accagaccca 1260

gtactttggg atcgtgggtg atgacaagag catgatgccg tcggataagg tgtgggaagg 1320
cggtgatgaa ggcgtcatgg ccaagcacag cagtgaggtg tttttcaaac cgggccaaga 1380
caacagcgtc agccgggccg cggtcgacgc ctccaaggaa tacttggagc acctggtccc 1440
tgaggagaag cgagagcgct ggggtgtcct ttcgaccgtg aagtcgctgg acatgtgcac 1500
agctgctggg ccaagctacg ggacagtgaa gcagcaggtc ttcaaccccg atggggcccc 1560
agtcaagcaa catgcggcca ccttttggcg tggggtgaac aacccgtgcg acggcaagtg 1620
tcggatctct ctgaaggatg agcttcggcc agcgaagaag cgtgagctgg gtctgacccg 1680
gcccatcttc tgttttgacg tgcatgacac ggtcagagtg aagagccaga ttgggtcggg 1740
cttgcaagcc ttggccgata cttgtgggac ccacatctgg agtgttggga tctcccaaca 1800
gaatggaact tggggtcagg tgtgtgagcg cctttccaag tggcgctacg ccatggatgc 1860
tgacttcggt cgttgggatt ccaccaactc acacccattg atgaagcagg cagctgaggt 1920
ccttgccagt ttggcgaccc gggaccaccg tgaggaagtg gaagaggact tgtcccggat 1980
gttgtcagcc caaacccagt ttggaccaac gatgaccggc cttccatccg ggttggtgtg 2040
cacggcacag atgaattgca cctcgcatct gttaaccatc aatgacatcc tgcttggcca 2100
cggtgcggcg cccgtaggct ccatgggctg ccctctggac tttgtgtcgt atggtgacga 2160
catcgtgttg gccatggacg accccaaagt cgcgacttgg ttgatcaatg gttggaaaaa 2220
gaggggattc caggcgacca gtgcggccaa gactggcccg ccgcaatgcg gcaagctcca 2280
ggacatgact ttcatcaagc gctccttcaa gaaggttgat ggtgagtggc gagccccgct 2340
ggaggcggcg tcgatttgga aggggttgag ctggatgcgt ggccatacct cctatgacca 2400
ctcagagggc gtccagaatg gagacctcac cggcactcgt gccgttggag tctttcagtc 2460
tgtgatgagc gagttctggc aacatggcaa ggaggtgttc gaggccgcca agaagctaat 2520
catcagctac gccagagaga ggaaattgcg gctcccggtg atcatcccgc catatgccca 2580
gttcaacccg aaggaggttt tggcagacta caatcgcctg gctggtgagt ccagcgtgtc 2640
catgctctcc gtttcctggc acacaggtgg cactggggaa gttcaaccag tcagccagac 2700
ggaaggtcca ggtggtgaga acgtcggaga agtcgttggc ctaccagcgt tggcgacagg 2760
cttggcacac cagaccgcgg ggattgagac cgccctggcc acctctggag gaccaactgt 2820
ccagctggat ccggcgaacc gtgagcggtt tgtgatggtg cctggtggca tggtgtctgt 2880
gcggacgaac atgacgcgcg gatccgttgt gtggcggaag aagatctcac ctgggatcaa 2940
catgtggacc aatctgctca gctccatgta caacgcctgg tgtggcggct tcgaggtcat 3000
gattgtggtt ggggccaata actttatcgg tgggaagttc ctggtggcct acttcccacc 3060
gaatgtggcg tctggaagct attcggtgga gcaggtgacg gctttccctc atgccattct 3120
ggacatccgt ctgatggaca acgtgcttct ggcctgctcg gacatcaagt acgtcctgtg 3180

gcacccgact aatgcggcgc ctgaggatcc gatcgcagtc gggggcgaag tggttgtcta 3240
cctgctgacg aacatcgtca cggctggcaa cagtggcaat gtgctgacgc tggacatgtc 3300
gatcttctca cgcccaatgc cggactttga cttcaacttt ctgatgccga tcagccttgg 3360
cggatctggt gggccaaacg acctggacat gcaggcggca ggtcgggcct tgtgcacacc 3420
agcaacggcc ggcggccgtg gatggcacgt gattgatgtt ctggtggcaa cccagcagtc 3480
gagcactgtg gctggggaca tgttcgcctc cacggtgagg atgagtggaa catcgcctta 3540
ctgcattccg tatccggctg cggatgggat gattgtggag gcaattaagg tgagcacaac 3600
ggattacaag ctcctgtgct acacccctga tgggacacca tgggatgttg ggaccgacaa 3660
ggcctcaaag actcttccac tctgctggat ggcctacaac tcagcgagcg accacacagt 3720
gctgacgtgg tacaagtctg atggcaactt ggagcacacc aatggcaaga tcaccctcgc 3780
ctccgtgggt gcgcagccgc attacgtccc atatgcggct gctgagattc ccgctgcgac 3840
tcggatcgcg gtgaccggca gcaaggatgg agctgccctg accaatcgtg agagcccggc 3900
gtccccgccg agcttcaccc ctaacaatgg ggaatccctg atcctgtacg ctggtctgcg 3960
caagacgggt cagaaggggc tgaccctctc cactcaggag atgatcacag tgttcatgga 4020
gtcaccgagg gtcacctcga tgtgtggact tttcaatgtg tccgatgcca ctggggcgac 4080
gactgtccag gccaaactct accctaacgg ggtgctgaca acagggacgg cggcgagcgc 4140
catctactac actggcccaa tttgcttcac ctacgttggg atggtcccag tggattacaa 4200
actgaatcct ccggctggag gaacgaatgc gaatgtcctg gatcttgttg aacggctgga 4260
caaatggcta gtgctgcagg agcgatcgca agtgggggcc tcagcgtcct cacaagcctc 4320
attgactcag caacttctct cggagtcgct gggatcaatc aaacgacggc ctttgaagtc 4380
caagctcgag actttggatt ccgacgagga atcattcgag accatactgc aagcttaggt 4440
caagctggcc tgcctggttt cttggcctat ggggggggttc atcccatgga ctactcgatc 4500
cgtatcgagt gtctcttgcg gctggctcgc acgtaggtcg ggcgtccatt gttaacaact 4560
cttttggaag cgtcccaaac cagttcggca atttccgcaa atctaacatg tctgctaccc 4620
cgcttgttaa gaagaagtag tcacgaccta taatcttgtt aaactcacat atactcagta 4680
agttttaaat gtagttgtat aaacatttta tagttatagt gttataatca tagattaatg 4740
catgccaaaa aaaaaaaaaa a 4761

<210> 2
<211> 7433
<212> DNA
<213> Salmonid calicivirus

<400> 2

cgaattattc gtattacaat tttcaaaaga acattaaaat gtctttttca aaactttgta 60

acaaaaataa cgagtctgtt attgactctg aactcaatac atcgagttca actttttccg 120
atcttagcaa gcggaatccg cttgctaata tcgactcttt gaaggaaggt gccaaggagg 180
tactcacagc agtgaaccaa atatttgacg tggttaaacc agtcactttg gctcacatca 240
tttgtgagat gctcgggcat acttcccgct ggaccattgt gcgctgtgtt gctcaactac 300
ttgagctcta caaaggtccg cttctttccc actggaagat catagtggat gcacttaaga 360
gtcttgctgc gctagttagt cgaaaggacg gtgacaccgt ccaagagcaa gaccccgagc 420
cggagcccaa aggggctacg ggcggtgaca agccgaaagc gtgtaaaacg gctttgtctg 480
ataagattgc acaagctgag acggcccccc cagatgagga ggccgtcaca gcgaaagcaa 540
agatggaggc ccatgttgat cgggccaagg aggagatgcg cggcaagttt gcgcgctgga 600
aggccgacgg cgatgcgctg ttggcaaata tgtcccaaat agggggcaag aacaagaaga 660
agaagaaggg ggcgattgat ctgacgctct ctggcccagt ccgggtgttt attgacacca 720
aagacgggga tctcttgagg tggtcttcag ctgcttttca ggcggctgaa gacttgggcg 780
cgaagtttgt ctacgcgcca tctgacaagg ctgaggtctt cgtcagtccc ctccacttca 840
cgtctcgtga ggcagctgag gttgacgtag ccaaggaaag tgagaagaag ggcacccggt 900
tgatcttttt ctttgggccg gctcacccca agctggatga cgtccctcac gaggagcgcg 960
tctccctgtt tggttggaag gtggtggact gcgccattgg tactggcaag aagaatgagg 1020
cccagagccg cgccttgacg gtctacttga atgagggtga ggggaagtgt gactgtgttg 1080
acaacgactg tgttcagtgg cacgctggtg acctccagga ctccatcttc tctgccctga 1140
aaggagctta cgcgagtagc gtcgaggtca tggttggctg ggttggcgcc ttacttgcgg 1200
gccttgtcgc cttggcgtct ctgttcccgg aaaacaagtt tgccaagaag gcggcagacc 1260
gcttcttggc actggcgagc aatgctggca agctgcatgc agcggccgat gacagtctgt 1320
acgcctacga tgcggtccag gggcccttta aggtcaccct tgccccagtt gcgtcgatga 1380
ctctgaagga tgctcactcc ttcatgaccg atgtgaagag cctactacag cggcgcccgc 1440
acatgctcac tcaccctgcc ttcccgatgg acagcatcaa tgccctgtac gactatgccg 1500
ccgcgcgtac tgggaagggt aaaagcattg gcgccaagtg ctgggcggac atcatgagcg 1560
attgttctat cgtccaacgc gctcacaagg atcgctttaa cacggcgacg acgaggccca 1620
tgccggtggt gttgtgcctg gcgggcccgc caggcgtagg gaagaccact gtcgtccggc 1680
aggttgccac tgagatcaac gagatcttcg ggaccgtcgg ctacgacgct tggcagtgtg 1740
gccttgacca ccaggaccag aacgccggca ggccagttgt gaccatggag gagttcgggg 1800
ctctggacct ctcacgtgac caagcggcgc tccagaggct ggcagacacc aaccctttg 1860
tgactgacaa tgacgtcatc cacaacaaag ggcgccacga agcaccagct gtcgtcatcg 1920

tgactaccaa ctgtcaagac atctacaccg gttttgccta ccccgacgct ctgtcgcgtc 1980

gagtcagcca ccacgttttg gtgaagaatg atggcctaga ggcctggaag acccggcacc 2040

cgaacaaggt gccaactgat cagaagtaca cagagatttt ctctgcgtgc ccgagcgtct 2100

actacaagtt gccggctgga gcttgtgact ggcaggggaa ttacctgcac aatggacttc 2160

aggggaacgt gccgcttaac accaaagcgt tggacgcgct ggttcgggac atcaaggcga 2220

cggtccgaaa ccgctggcgc aacgtgctcg aggatgagac tgagatgcct gggaagctag 2280

tcttccacgc caaggacgtg cccgttatgg ttttccgagg cccacctggc tgcgggaaga 2340

ccaccttggc cgggtgcgtc gctggccagt ttgaggtgat taacgacagc tggaccaaga 2400

aggacgcgtt caagaccgtg gttgaccatg tcatgcatct ggaggagcac gctgacccca 2460

ccccggtgtt gatcaccacc aacatcaacc cctggcggga tgagcttgcc aagatgccca 2520

aggagcaacg ccttgcgttt gagcgtcggg tgaagtatta caacttctcc tacaagaaga 2580

agaactttct gacgcactac acccatgcgg acatgaagga ctgtgggtgg tccaagatcg 2640

ttgacgtggt ggggccggat ggatcccatt atacgtacac caccctggtt gaggaactgc 2700

gcgcggccgc gccagttcag aaggctgtcg ctgaggagga cgccccgatc cagaacgagg 2760

cgtacgcctg cgcggctgag acgaacatga gtgttgccca ggttggcgct ggcgaggacc 2820

cgttgaaggt tttgctgtcg ttcgagagac acaacgttcc ggggctgaac atgctgacct 2880

ccttctctag tgtggtcaag cagattcaga tccagggcaa gactcggacc catcccaaat 2940

ctctggtgtg ctccatcaac gacgctaagt tggtgctcaa gatcccgcca tgcagcgtca 3000

agtttgcgga tgccgtcgtg aacttctgga gctacgaggg caagttgcgg tgtgccctag 3060

tcaaggaggg tgtcaaagag agtgccgggt tgggtttctc cacctcaccc agcgtggacc 3120

cgtttgcctt cgaccaagtg gtcgaagatg agtacgagct gcccaagtct atcagtgggg 3180

cgctgctgaa tgtgatgacc accgtgctcg ggatggtggc gatggcgatg gaagccaaag 3240

tcactttcca cacaccgacc gaagaggatg agaaaggacc agctcgacgg gtgactcgat 3300

cttaccgacc cgtacgggac agtgacttcc agccagctgg tcgcctgtgg gcggaagatc 3360

ggcgcgaaat gggctacacc gaacggatta cgttcgattc caataactgg atcgtcccct 3420

gcttcgacgc ggctgggtgc ccaaaaggtt gggcgatctc tacgcccaag ggcctgctgg 3480

cgaaccggca tgtgatcgct gggacgacta ggcttggcca ccaaaaagtt ggcgagtccg 3540

tggtcctcaa ctacggcgac actgaccttt gcctgatcaa gaacaccaag aacccatacc 3600

agctgtgtaa ggcgcctttc tcccgaccca ttgttggtga gatcctttac cagctgaagc 3660

catctgggat gcagcgggct cgtgtccaca gtttcgtccg ggtggccaac ccaagtggca 3720

agctcgttga gatgtgcctg gtcgaagggg gcgtcagtga ccctggtgat tgtggcctgc 3780

catgggtccg ccgcgtgggc cgaagtgtgg agttggtcgg cctggcagcc ggcactcgag 3840

gggcgaggct gatggtcgtt ccactggggg ttgacccagc tgagacccag tggcatgcca 3900

gaacccacaa ctacaccttt ctgcaccaga cccagtactt tgggatcgtg ggtgatgaca 3960

agagcatgat gccgtcggat aaggtgtggg aaggcggtga tgaaggcgtc atggccaagc 4020

acagcagtga ggtgtttttc aaaccgggcc aagacaacag cgtcagccgg gccgcggtcg 4080

acgcctccaa ggaatacttg gagcacctgg tccctgagga gaagcgagag cgctggggtg 4140

tcctttcgac cgtgaagtcg ctggacatgt gcacagctgc tgggccaagc tacgggacag 4200

tgaagcagca ggtcttcaac cccgatgggg ccccagtcaa gcaacatgcg gccacctttt 4260

ggcgtggggt gaacaacccg tgcgacggca agtgtcggat ctctctgaag gatgagcttc 4320

ggccagcgaa gaagcgtgag ctgggtctga cccggcccat cttctgtttt gacgtgcatg 4380

acacggtcag agtgaagagc cagattgggt cgggcttgca agccttggcc gatacttgtg 4440

ggacccacat ctggagtgtt gggatctccc aacagaatgg aacttggggt caggtgtgtg 4500

agcgcctttc caagtggcgc tacgccatgg atgctgactt cggtcgttgg gattccacca 4560

actcacaccc attgatgaag caggcagctg aggtccttgc cagtttggcg acccgggacc 4620

accgtgagga agtggaagag gacttgtccc ggatgttgtc agcccaaacc cagtttggac 4680

caacgatgac cggccttcca tccgggttgg tgtgcacggc acagatgaat tgcacctcgc 4740

atctgttaac catcaatgac atcctgcttg gccacggtgc ggcgcccgta ggctccatgg 4800

gctgccctct ggactttgtg tcgtatggtg acgacatcgt gttggccatg gacgacccca 4860

aagtcgcgac ttggttgatc aatggttgga aaaagagggg attccaggcg accagtgcgg 4920

ccaagactgg cccgccgcaa tgcggcaagc tccaggacat gactttcatc aagcgctcct 4980

tcaagaaggt tgatggtgag tggcgagccc cgctggaggc ggcgtcgatt tggaaggggt 5040

tgagctggat gcgtggccat acctcctatg accactcaga gggcgtccag aatggagacc 5100

tcaccggcac tcgtgccgtt ggagtctttc agtctgtgat gagcgagttc tggcaacatg 5160

gcaaggaggt gttcgaggcc gccaagaagc taatcatcag ctacgccaga gagaggaaat 5220

tgcggctccc ggtgatcatc ccgccatatg cccagttcaa cccgaaggag gttttggcag 5280

actacaatcg cctggctggt gagtccagcg tgtccatgct ctccgtttcc tggcacacag 5340

gtggcactgg ggaagttcaa ccagtcagcc agacggaagg tccaggtggt gagaacgtcg 5400

gagaagtcgt tggcctacca gcgttggcga caggcttggc acaccagacc gcggggattg 5460

agaccgccct ggccacctct ggaggaccaa ctgtccagct ggatccggcg aaccgtgagc 5520

ggtttgtgat ggtgcctggt ggcatggtgt ctgtgcggac gaacatgacg cgcggatccg 5580

ttgtgtggcg gaagaagatc tcacctggga tcaacatgtg gaccaatctg ctcagctcca 5640

tgtacaacgc ctggtgtggc ggcttcgagg tcatgattgt ggttggggcc aataacttta 5700

tcggtgggaa gttcctggtg gcctacttcc caccgaatgt ggcgtctgga agctattcgg 5760
tggagcaggt gacggctttc cctcatgcca ttctggacat ccgtctgatg gacaacgtgc 5820
ttctggcctg ctcggacatc aagtacgtcc tgtggcaccc gactaatgcg gcgcctgagg 5880
atccgatcgc agtcgggggc gaagtggttg tctacctgct gacgaacatc gtcacggctg 5940
gcaacagtgg caatgtgctg acgctggaca tgtcgatctt ctcacgccca atgccggact 6000
ttgacttcaa ctttctgatg ccgatcagcc ttggcggatc tggtgggcca aacgacctgg 6060
acatgcaggc ggcaggtcgg gccttgtgca caccagcaac ggccggcggc cgtggatggc 6120
acgtgattga tgttctggtg gcaacccagc agtcgagcac tgtggctggg gacatgttcg 6180
cctccacggt gaggatgagt ggaacatcgc cttactgcat tccgtatccg gctgcggatg 6240
ggatgattgt ggaggcaatt aaggtgagca caacggatta caagctcctg tgctacaccc 6300
ctgatgggac accatgggat gttgggaccg acaaggcctc aaagactctt ccactctgct 6360
ggatggccta caactcagcg agcgaccaca cagtgctgac gtggtacaag tctgatggca 6420
acttggagca caccaatggc aagatcaccc tcgcctccgt gggtgcgcag ccgcattacg 6480
tcccatatgc ggctgctgag attcccgctg cgactcggat cgcggtgacc ggcagcaagg 6540
atggagctgc cctgaccaat cgtgagagcc cggcgtcccc gccgagcttc accccctaaca 6600
atggggaatc cctgatcctg tacgctggtc tgcgcaagac gggtcagaag gggctgaccc 6660
tctccactca ggagatgatc acagtgttca tggagtcacc gagggtcacc tcgatgtgtg 6720
gacttttcaa tgtgtccgat gccactgggg cgacgactgt ccaggccaaa ctctacccta 6780
acggggtgct gacaacaggg acggcggcga gcgccatcta ctacactggc ccaatttgct 6840
tcacctacgt tgggatggtc ccagtggatt acaaactgaa tcctccggct ggaggaacga 6900
atgcgaatgt cctggatctt gttgaacggc tggacaaatg gctagtgctg caggagcgat 6960
cgcaagtggg ggcctcagcg tcctcacaag cctcattgac tcagcaactt ctctcggagt 7020
cgctgggatc aatcaaacga cggcctttga agtccaagct cgagactttg gattccgacg 7080
aggaatcatt cgagaccata ctgcaagctt aggtcaagct ggcctgcctg gtttcttggc 7140
ctatgggggt tcatcccatg gactactcga tccgtatcga gtgtctcttg cggctggctc 7200
gcacgtaggt cgggcgtcca ttgttaacaa ctcttttgga agcgtcccaa accagttcgg 7260
caatttccgc aaatctaaca tgtctgctac cccgcttgtt aagaagaagt agtcacgacc 7320
tataatcttg ttaaactcac atatactcag taagttttaa atgtagttgt ataaacattt 7380
tatagttata gtgttataat catagattaa tgcatgccaa aaaaaaaaaa aaa 7433

<210> 3
<211> 4761
<212> RNA

<213> Salmonid calicivirus

<400> 3

guacaccacc cugguugagg aacugcgcgc ggccgcgcca guucagaagg cugucgcuga 60
ggaggacgcc ccgauccaga acgaggcgua cgccugcgcg gcugagacga acaugagugu 120
ugcccagguu ggcgcuggcg aggacccguu gaagguuuug cugucguucg agagacacaa 180
cguuccgggg cugaacaugc ugaccuccuu cucuagugug gucaagcaga uucagaucca 240
gggcaagacu cggacccauc ccaaaucucu ggugugcucc aucaacgacg cuaaguuggu 300
gcucaagauc ccgccaugca gcgucaaguu ugcggaugcc gucgugaacu ucuggagcua 360
cgagggcaag uugcggugug cccuagucaa ggagggugug aaagagagug ccggguuggg 420
uuucuccacc ucacccagcg uggacccguu ugccuucgac caaguggucg aagaugagua 480
cgagcugccc aagucuauca guggggcgcu gcugaaugug augaccaccg ugcucgggau 540
gguggcgaug gcgauggaag ccaaagucac uuuccacaca ccgaccgaag aggaugagaa 600
aggaccagcu cgacggguga cucgaucuua ccgacccgua cgggacagug acuuccagcc 660
agcuggucgc cugugggcgg aagaucggcg cgaaaugggc uacaccgaac ggauuacguu 720
cgauuccaau aacuggaucg uccccugcuu cgacgcggcu gggugcccaa aagguugggc 780
gaucucuacg cccaagggcc ugcuggcgaa ccggcaugug aucgcuggga cgacuaggcu 840
uggccaccaa aaaguuggcg aguccguggu ccucaacuac ggcgacacug accuuugccu 900
gaucaagaac accaagaacc cauaccagcu guguaaggcg ccuuucuccc gacccauugu 960
uggugagauc cuuuaccagc ugaagccauc ugggaugcag cgggcucgug uccacaguuu 1020
cguccgggug gccaacccaa guggcaagcu cguugagaug ugccuggucg aagggggcgu 1080
cagugacccu ggugauugug gccugccaug gguccgccgc gugggccgaa guguggaguu 1140
ggucggccug gcagccggca cucgaggggc gaggcugaug gucguuccac ugggggguuga 1200
cccagcugag acccaguggc augccagaac ccacaacuac accuuucugc accagaccca 1260
guacuuuggg aucgugggug augacaagag caugaugccg ucggauaagg ugugggaagg 1320
cggugaugaa ggcgucaugg ccaagcacag cagugaggug uuuuucaaac cgggccaaga 1380
caacagcguc agccgggccg cggucgacgc cuccaaggaa uacuuggagc accugguccc 1440
ugaggagaag cgagagcgcu ggggguuccu uucgaccgug aagucgcugg acaugugcac 1500
agcugcuggg ccaagcuacg ggacagugaa gcagcagguc uucaaccccg augggggcccc 1560
agucaagcaa caugcggcca ccuuuuggcg uggggugaac aacccgugcg acggcaagug 1620
ucggaucucu cugaaggaug agcuucggcc agcgaagaag cgugagcugg gucugacccg 1680
gcccaucuuc uguuuugacg ugcaugacac ggucagagug aagagccaga uugggucggg 1740
cuugcaagcc uuggccgaua cuugugggac ccacaucugg aguguuggga ucucccaaca 1800
gaauggaacu uggggucagg ugugugagcg ccuuuccaag uggcgcuacg ccauggaugc 1860

ugacuucggu cguugggauu ccaccaacuc acacccauug augaagcagg cagcugaggu 1920
ccuugccagu uuggcgaccc gggaccaccg ugaggaagug gaagaggacu ugucccggau 1980
guugucagcc caaacccagu uuggaccaac gaugaccggc cuuccauccg gguuggugug 2040
cacggcacag augaauugca ccucgcaucu guuaaccauc aaugacaucc ugcuuggcca 2100
cggugcggcg cccguaggcu ccaugggcug cccucuggac uuugugucgu auggugacga 2160
caucguguug gccauggacg accccaaagu cgcgacuugg uugaucaaug guuggaaaaa 2220
gaggggauuc caggcgacca gugcggccaa gacuggcccg ccgcaaugcg gcaagcucca 2280
ggacaugacu uucaucaagc gcuccuucaa gaagguugau ggugaguggc gagccccgcu 2340
ggaggcggcg ucgauuugga aggggaugag cuggaugcgu ggccauaccu ccuaugacca 2400
cucagagggc guccagaaug gagaccucac cggcacucgu gccguuggag ucuuucaguc 2460
ugugaugagc gaguucuggc aacauggcaa ggagguguuc gaggccgcca agaagcuaau 2520
caucagcuac gccagagaga ggaaauugcg gcucccggug aucaucccgc cauaugccca 2580
guucaacccg aaggagguuu uggcagacua caaucgccug gcuggugagu ccagcguguc 2640
caugcucucc guuuccuggc acacaggugg cacuggggaa guucaaccag ucagccagac 2700
ggaaggucca gguggugaga acgucggaga agucguuggc cuaccagcgu uggcgacagg 2760
cuuggcacac cagaccgcgg ggauugagac cgcccuggcc accucuggag gaccaacugu 2820
ccagcuggau ccggcgaacc gugagcgguu ugugauggug ccugguggca uggugucugu 2880
gcggacgaac augacgcgcg gauccguugu guggcggaag aagaucucac cugggaucaa 2940
cauguggacc aaucugcuca gcuccaugua caacgccugg uguggcggcu ucgaggucau 3000
gauugugguu ggggccaaua acuuuaucgg ugggaaguuc cugguggccu acuucccacc 3060
gaauguggcg ucuggaagcu auucggugga gcaggugacg gcuuucccuc augccauucu 3120
ggacauccgu cugauggaca acgugcuucu ggccugcucg gacaucaagu acguccugug 3180
gcacccgacu aaugcggcgc cugaggaucc gaucgcaguc gggggcgaag ugguugucua 3240
ccugcugacg aacaucguca cggcuggcaa caguggcaau gugcugacgc uggacauguc 3300
gaucuucuca cgcccaaugc cggacuuuga cuucaacuuu cugaugccga ucagccuugg 3360
cggaucuggu gggccaaacg accuggacau gcaggcggca ggucgggccu ugugcacacc 3420
agcaacggcc ggcggccgug gauggcacgu gauugauguu cugguggcaa cccagcaguc 3480
gagcacugug gcuggggaca uguucgccuc cacggugagg augaguggaa caucgccuua 3540
cugcauuccg uauccggcug cggaugggau gauuguggag gcaauuaagg ugagcacaac 3600
ggauuacaag cuccugugcu acaccccuga ugggacacca ugggauguug ggaccgacaa 3660
ggccucaaag acucuuccac ucugcuggau ggccuacaac ucagcgagcg accacacagu 3720

gcugacgugg uacaagucug auggcaacuu ggagcacacc aauggcaaga ucacccucgc 3780

cuccgugggu gcgcagccgc auuacguccc auaugcggcu gcugagauuc ccgcugcgac 3840

ucggaucgcg gugaccggca gcaaggaugg agcugcccug accaaucgug agagcccggc 3900

guccccgccg agcuucaccc cuaacaaugg ggaaucccug auccuguacg cuggucugcg 3960

caagacgggu cagaaggggc ugacccucuc cacucaggag augaucacag uguucaugga 4020

gucaccgagg gucaccucga ugugugggacu uuucaaugug uccgaugcca cuggggcgac 4080

gacuguccag gccaaacucu acccuaacgg ggugcugaca acagggacgg cggcgagcgc 4140

caucuacuac acuggcccaa uuugcuucac cuacguuggg augguccag uggauuacaa 4200

acugaauccu ccggcuggag gaacgaaugc gaauguccug gaucuuguug aacggcugga 4260

caaauggcua gugcugcagg agcgaucgca aguggggggcc ucagcguccu cacaagccuc 4320

auugacucag caacuucucu cggagucgcu gggaucaauc aaacgacggc cuuugaaguc 4380

caagcucgag acuuuggauu ccgacgagga aucauucgag accauacugc aagcuuaggu 4440

caagcuggcc ugccugguuu cuuggccuau gggggggguuc aucccaugga cuacucgauc 4500

cguaucgagu gucucuugcg gcuggcucgc acguaggucg ggcguccauu guuaacaacu 4560

cuuuuggaag cgucccaaac caguucggca auuuccgcaa aucuaacaug ucugcuaccc 4620

cgcuuguuaa gaagaaguag ucacgaccua uaaucuuguu aaacucacau auacucagua 4680

aguuuuaaau guaguuguau aaacauuuua uaguuauagu guuauaauca uagauuaaug 4740

caugccaaaa aaaaaaaaaa a 4761

<210> 4
<211> 7433
<212> RNA
<213> Salmonid calicivirus

<400> 4

```
cgaauuauuc guauuacaau uuucaaaaga acauuaaaau gucuuuuuca aaacuuugua        60
acaaaaauaa cgagucuguu auugacucug aacucaauac aucgaguuca acuuuuuccg       120
aucuuagcaa gcggaauccg cuugcuaaua ucgacucuuu gaaggaaggu gccaaggagg       180
uacucacagc agugaaccaa auauuugacg ugguuaaacc agucacuuug gcucacauca       240
uuugugagau gcucgggcau acuuccccgcu ggaccauugu gcgcuguguu gcucaacuac      300
uugagcucua caaagguccg cuucuuuccc acuggaagau cauaguggau gcacuuaaga       360
gucuugcugc gcuaguuagu cgaaaggacg gugacaccgu ccaagagcaa gaccccgagc       420
cggagcccaa aggggcuacg ggcggugaca agccgaaagc guguaaaacg gcuuugucug       480
auaagauugc acaagcugag acggcccccc cagaugagga ggccgucaca gcgaaagcaa       540
agauggaggc ccauguugau cgggccaagg aggagaugcg cggcaaguuu gcgcgcugga       600
```

aggccgacgg cgaugcgcug uuggcaaaua ugucccaaau agggggcaag aacaagaaga 660
agaagaaggg ggcgauugau cugacgcucu cuggcccagu ccggguguuu auugacacca 720
aagacgggga ucucuugagg uggucuucag cugcuuuuca ggcggcugaa gacuugggcg 780
cgaaguuugu cuacgcgcca ucugacaagg cugaggucuu cgucaguccc cuccacuuca 840
cgucucguga ggcagcugag guugacguag ccaaggaaag ugagaagaag ggcacccggu 900
ugaucuuuuu cuuugggccg gcucacccca agcuggauga cgucccucac gaggagcgcg 960
ucucccuguu ugguuggaag gugguggacu gcgccauugg uacuggcaag aagaaugagg 1020
cccagagccg cgccuugacg gucuacuuga augaggguga ggggaagugu gacuguguug 1080
acaacgacug uguucagugg cacgcuggug accuccagga cuccaucuuc ucugcccuga 1140
aaggagcuua cgcgaguagc gucgagguca ugguuggcug gguuggcgcc uuacuugcgg 1200
gccuugucgc cuuggcgucu cuguucccgg aaaacaaguu ugccaagaag gcggcagacc 1260
gcuucuuggc acuggcgagc aaugcuggca agcugcaugc agcggccgau gacagucugu 1320
acgccuacga ugcgguccag gggcccuua agggucacccu ugccccaguu gcgucgauga 1380
cucugaagga ugcucacucc uucaugaccg augugaagag ccuacuacag cggcgcccgc 1440
acaugcucac ucacccugcc uucccgaugg acagcaucaa ugcccuguac gacuaugccg 1500
ccgcgcguac ugggaagggu aaaagcauug gcgccaagug cugggcggac aucaugagcg 1560
auuguucuau cguccaacgc gcucacaagg aucgcuuuaa cacggcgacg acgaggccca 1620
ugccggugggu guugugccug gcgggcccgc caggcguagg gaagaccacu gucguccggc 1680
agguugccac ugagaucaac gagaucuucg ggaccgucgg cuacgacgcu uggcagugug 1740
gccuugacca ccaggaccag aacgccggca ggccaguugu gaccauggag gaguucgggg 1800
cucuggaccu cucacgugac caagcggcgc uccagaggcu ggcagacacc aacccuuuug 1860
ugacugacaa ugacgucauc cacaacaaag ggcgccacga agcaccagcu gucgucaucg 1920
ugacuaccaa cugucaagac aucuacaccg guuuugccua ccccgacgcu cugucgcguc 1980
gagucagcca ccacguuuug gugaagaaug auggccuaga ggccuggaag acccggcacc 2040
cgaacaaggu gccaacugau cagaaguaca cagagauuuu cucugcgugc ccgagcgucu 2100
acuacaaguu gccggcugga gcuugugacu ggcaggggaa uuaccugcac aauggacuuc 2160
aggggaacgu gccgcuuaac accaaagcgu uggacgcgcu gguucgggac aucaaggcga 2220
cgguccgaaa ccgcuggcgc aacgugcucg aggaugagac ugagaugccu gggaagcuag 2280
ucuuccacgc caaggacgug cccguuaugg uuuuccgagg cccaccuggc ugcgggaaga 2340
ccaccuuggc cgggugcguc gcuggccagu uugaggugau uaacgacagc uggaccaaga 2400
aggacgcguu caagaccgug guugaccaug ucaugcaucu ggaggagcac gcugacccca 2460
ccccggguguu gaucaccacc aacaucaacc ccuggcggga ugagcuugcc aagaugccca 2520

aggagcaacg ccuugcguuu gagcgucggg ugaaguauua caacuucucc uacaagaaga 2580
agaacuuucu gacgcacuac acccaugcgg acaugaagga cugugggugg uccaagaucg 2640
uugacguggu ggggccggau ggaucccauu auacguacac cacccugguu gaggaacugc 2700
gcgcggccgc gccaguucag aaggcugucg cugaggagga cgccccgauc cagaacgagg 2760
cguacgccug cgcggcugag acgaacauga guguugccca gguuggcgcu ggcgaggacc 2820
cguugaaggu uuugcugucg uucgagagac acaacguucc ggggcugaac augcugaccu 2880
ccuucucuag uguggucaag cagauucaga uccagggcaa gacucggacc caucccaaau 2940
cucuggugug cuccaucaac gacgcuaagu uggugcucaa gaucccgcca ugcagcguca 3000
aguuugcgga ugccgucgug aacuucugga gcuacgaggg caaguugcgg ugugcccuag 3060
ucaaggaggg ugucaaagag agugccgggu uggguuucuc caccucaccc agcguggacc 3120
cguuugccuu cgaccaagug gucgaagaug aguacgagcu gcccaagucu aucagugggg 3180
cgcugcugaa ugugaugacc accgugcucg ggauggugge gauggcgaug gaagccaaag 3240
ucacuuucca cacaccgacc gaagaggaug agaaaggacc agcucgacgg gugacucgau 3300
cuuaccgacc cguacgggac agugacuucc agccagcugg ucgccugugg gcggaagauc 3360
ggcgcgaaau gggcuacacc gaacggauua cguucgauuc caauaacugg aucgucccu 3420
gcuucgacgc ggcugggugc ccaaaagguu gggcgaucuc uacgcccaag ggccugcugg 3480
cgaaccggca ugugaucgcu gggacgacua ggcuuggcca ccaaaaaguu ggcgaguccg 3540
ugguccucaa cuacggcgac acugaccuuu gccugaucaa gaacaccaag aacccauacc 3600
agcuguguaa ggcgccuuuc ucccgaccca uuguugguga gauccuuuac cagcugaagc 3660
caucugggau gcagcgggcu cguguccaca guuucguccg gguggccaac ccaaguggca 3720
agcucguuga gaugugccug gucgaagggg gcgucaguga cccuggugau uguggccugc 3780
caugggucca ccgcgugggc cgaagugugg aguuggucgg ccuggcagcc ggcacucgag 3840
gggcgaggcu gauggucguu ccacuggggg uugacccagc ugagacccag uggcaugcca 3900
gaacccacaa cuacaccuuu cugcaccaga cccaguacuu ugggaucgug ggugaugaca 3960
agagcaugau gccgucggau aaggugugg aaggcgguga ugaaggcguc auggccaagc 4020
acagcaguga gguguuuuuc aaaccgggcc aagacaacag cgucagccgg gccgcggucg 4080
acgccuccaa ggaauacuug gagcaccugg ucccugagga gaagcgagag cgcuggggug 4140
uccuuucgac cgugaagucg cuggacaugu gcacagcugc ugggccaagc uacgggacag 4200
ugaagcagca ggucuucaac cccgaugggg ccccagucaa gcaacaugcg gccaccuuuu 4260
ggcguggggu gaacaacccg ugcgacggca agugucggau cucucugaag gaugagcuuc 4320
ggccagcgaa gaagcgugag cugggucuga cccggcccau cuucuguuuu gacgugcaug 4380

acacggucag agugaagagc cagauugggu cgggcuugca agccuuggcc gauacuugug 4440

ggacccacau cuggaguguu gggaucuccc aacagaaugg aacuuggggu caggugugug 4500

agcgccuuuc caaguggcgc uacgccaugg augcugacuu cggucguugg gauuccacca 4560

acucacaccc auugaugaag caggcagcug agguccuugc caguuuggcg acccgggacc 4620

accgugagga aguggaagag gacuuguccc ggauguuguc agcccaaacc caguuuggac 4680

caacgaugac cggccuucca uccggguugg ugugcacggc acagaugaau ugcaccucgc 4740

aucuguuaac caucaaugac auccugcuug gccacggugc ggcgcccgua ggcuccaugg 4800

gcugcccucu ggacuuugug ucguauggug acgacaucgu guuggccaug gacgacccca 4860

aagucgcgac uugguugauc aaugguugga aaaagagggg auuccaggcg accagugcgg 4920

ccaagacugg cccgccgcaa ugcggcaagc uccaggacau gacuuucauc aagcgcuccu 4980

ucaagaaggu ugauggugag uggcgagccc cgcuggaggc ggcgucgauu uggaaggggu 5040

ugagcuggau gcguggccau accuccuaug accacucaga gggcguccag aauggagacc 5100

ucaccggcac ucgugccguu ggagucuuuc agucugugau gagcgaguuc uggcaacaug 5160

gcaaggaggu guucgaggcc gccaagaagc uaaucaucag cuacgccaga gagaggaaau 5220

ugcggcuccc ggugaucauc ccgccauaug cccaguucaa cccgaaggag guuuuggcag 5280

acuacaaucg ccuggcuggu gaguccagcg uguccaugcu cuccguuucc uggcacacag 5340

guggcacugg ggaaguucaa ccagucagcc agacggaagg uccagguggu gagaacgucg 5400

gagaagucgu uggccuacca gcguuggcga caggcuuggc acaccagacc gcggggauug 5460

agaccgcccu ggccaccucu ggaggaccaa cuguccagcu ggauccggcg aaccgugagc 5520

gguuugugau ggugccuggu ggcauggugu cugugcggac gaacaugacg cgcggauccg 5580

uuguguggcg gaagaagauc ucaccuggga ucaacaugug gaccaaucug cucagcucca 5640

uguacaacgc cugguguggc ggcuucgagg ucaugauugu gguuggggcc aauaacuuua 5700

ucggugggaa guuccuggug gccuacuucc caccgaaugu ggcgucugga agcuauucgg 5760

uggagcaggu gacggcuuuc ccucaugcca uucuggacau ccgucugaug gacaacgugc 5820

uucuggccug cucggacauc aaguacgucc uguggcaccc gacuaaugcg gcgccugagg 5880

auccgaucgc agucgggggc gaagugguug ucuaccugcu gacgaacauc gucacggcug 5940

gcaacagugg caaugugcug acgcuggaca ugucgaucuu cucacgccca augccggacu 6000

uugacuucaa cuuucugaug ccgaucagcc uuggcggauc ugguggggcca aacgaccugg 6060

acaugcaggc ggcaggucgg gccuugugca caccagcaac ggccggcggc cguggauggc 6120

acgugauuga uguucuggug gcaacccagc agucgagcac uguggcuggg gacauguucg 6180

ccuccacggu gaggaugagu ggaacaucgc cuuacugcau uccguauccg gcugcggaug 6240

ggaugauugu ggaggcaauu aaggugagca caacggauua caagcuccug ugcuacaccc 6300

cugaugggac accaugggau guugggaccg acaaggccuc aaagacucuu ccacucugcu 6360

ggauggccua caacucagcg agcgaccaca cagugcugac gugguacaag ucugauggca 6420

acuuggagca caccaauggc aagaucaccc ucgccuccgu gggugcgcag ccgcauuacg 6480

ucccauaugc ggcugcugag auucccgcug cgacucggau cgcggugacc ggcagcaagg 6540

auggagcugc ccugaccaau cgugagagcc cggcgucccc gccgagcuuc accccuaaca 6600

auggggaauc ccugauccug uacgcugguc ugcgcaagac gggucagaag gggcugaccc 6660

ucuccacuca ggagaugauc acaguguuca uggagucacc gagggucacc ucgaugugug 6720

gacuuuucaa uguguccgau gccacugggg cgacgacugu ccaggccaaa cucuacccua 6780

acggggugcu gacaacaggg acggcggcga gcgccaucua cuacacuggc ccaauuugcu 6840

ucaccuacgu ugggauggu c ccaguggauu acaaacugaa ucuccggcu ggaggaacga 6900

augcgaaugu ccuggaucuu guugaacggc uggacaaaug gcuagugcug caggagcgau 6960

cgcaaguggg ggccucagcg uccucacaag ccucauugac ucagcaacuu cucucggagu 7020

cgcugggauc aaucaaacga cggccuuuga aguccaagcu cgagacuuug gauuccgacg 7080

aggaaucauu cgagaccaua cugcaagcuu aggucaagcu ggccugccug guuucuuggc 7140

cuauggggu ucaucccaug gacuacucga uccguaucga gugucucuug cggcuggcuc 7200

gcacguaggu cgggcgucca uuguuaacaa cucuuuugga agcgucccaa accaguucgg 7260

caauuuccgc aaaucuaaca ugucugcuac cccgcuuguu aagaagaagu agucacgacc 7320

uauaaucuug uuaaacucac auauacucag uaaguuuuaa auguaguugu auaaacauuu 7380

uauaguuaua guguuauaau cauagauuaa ugcaugccaa aaaaaaaaaa aaa 7433

<210> 5
<211> 1441
<212> PRT
<213> Salmonid calicivirus

<400> 5

Met Ser Val Ala Gln Val Gly Ala Gly Glu Asp Pro Leu Lys Val Leu
1 5 10 15

Leu Ser Phe Glu Arg His Asn Val Pro Gly Leu Asn Met Leu Thr Ser
20 25 30

Phe Ser Ser Val Val Lys Gln Ile Gln Ile Gln Gly Lys Thr Arg Thr
35 40 45

His Pro Lys Ser Leu Val Cys Ser Ile Asn Asp Ala Lys Leu Val Leu
50 55 60

```
Lys Ile Pro Pro Cys Ser Val Lys Phe Ala Asp Ala Val Val Asn Phe
65                  70                  75                  80

Trp Ser Tyr Glu Gly Lys Leu Arg Cys Ala Leu Val Lys Glu Gly Val
                85                  90                  95

Lys Glu Ser Ala Gly Leu Gly Phe Ser Thr Ser Pro Ser Val Asp Pro
            100                 105                 110

Phe Ala Phe Asp Gln Val Val Glu Asp Glu Tyr Glu Leu Pro Lys Ser
        115                 120                 125

Ile Ser Gly Ala Leu Leu Asn Val Met Thr Thr Val Leu Gly Met Val
    130                 135                 140

Ala Met Ala Met Glu Ala Lys Val Thr Phe His Thr Pro Thr Glu Glu
145                 150                 155                 160

Asp Glu Lys Gly Pro Ala Arg Arg Val Thr Arg Ser Tyr Arg Pro Val
                165                 170                 175

Arg Asp Ser Asp Phe Gln Pro Ala Gly Arg Leu Trp Ala Glu Asp Arg
            180                 185                 190

Arg Glu Met Gly Tyr Thr Glu Arg Ile Thr Phe Asp Ser Asn Asn Trp
        195                 200                 205

Ile Val Pro Cys Phe Asp Ala Ala Gly Cys Pro Lys Gly Trp Ala Ile
    210                 215                 220

Ser Thr Pro Lys Gly Leu Leu Ala Asn Arg His Val Ile Ala Gly Thr
225                 230                 235                 240

Thr Arg Leu Gly His Gln Lys Val Gly Glu Ser Val Val Leu Asn Tyr
                245                 250                 255

Gly Asp Thr Asp Leu Cys Leu Ile Lys Asn Thr Lys Asn Pro Tyr Gln
            260                 265                 270

Leu Cys Lys Ala Pro Phe Ser Arg Pro Ile Val Gly Glu Ile Leu Tyr
        275                 280                 285

Gln Leu Lys Pro Ser Gly Met Gln Arg Ala Arg Val His Ser Phe Val
    290                 295                 300

Arg Val Ala Asn Pro Ser Gly Lys Leu Val Glu Met Cys Leu Val Glu
305                 310                 315                 320
```

```
Gly Gly Val Ser Asp Pro Gly Asp Cys Gly Leu Pro Trp Val Arg Arg
                325                 330                 335

Val Gly Arg Ser Val Glu Leu Val Gly Leu Ala Ala Gly Thr Arg Gly
            340                 345                 350

Ala Arg Leu Met Val Val Pro Leu Gly Val Asp Pro Ala Glu Thr Gln
        355                 360                 365

Trp His Ala Arg Thr His Asn Tyr Thr Phe Leu His Gln Thr Gln Tyr
    370                 375                 380

Phe Gly Ile Val Gly Asp Asp Lys Ser Met Met Pro Ser Asp Lys Val
385                 390                 395                 400

Trp Glu Gly Gly Asp Glu Gly Val Met Ala Lys His Ser Ser Glu Val
                405                 410                 415

Phe Phe Lys Pro Gly Gln Asp Asn Ser Val Ser Arg Ala Ala Val Asp
            420                 425                 430

Ala Ser Lys Glu Tyr Leu Glu His Leu Val Pro Glu Glu Lys Arg Glu
        435                 440                 445

Arg Trp Gly Val Leu Ser Thr Val Lys Ser Leu Asp Met Cys Thr Ala
    450                 455                 460

Ala Gly Pro Ser Tyr Gly Thr Val Lys Gln Gln Val Phe Asn Pro Asp
465                 470                 475                 480

Gly Ala Pro Val Lys Gln His Ala Ala Thr Phe Trp Arg Gly Val Asn
                485                 490                 495

Asn Pro Cys Asp Gly Lys Cys Arg Ile Ser Leu Lys Asp Glu Leu Arg
            500                 505                 510

Pro Ala Lys Lys Arg Glu Leu Gly Leu Thr Arg Pro Ile Phe Cys Phe
        515                 520                 525

Asp Val His Asp Thr Val Arg Val Lys Ser Gln Ile Gly Ser Gly Leu
    530                 535                 540

Gln Ala Leu Ala Asp Thr Cys Gly Thr His Ile Trp Ser Val Gly Ile
545                 550                 555                 560

Ser Gln Gln Asn Gly Thr Trp Gly Gln Val Cys Glu Arg Leu Ser Lys
                565                 570                 575
```

Trp Arg Tyr Ala Met Asp Ala Asp Phe Gly Arg Trp Asp Ser Thr Asn
580 585 590

Ser His Pro Leu Met Lys Gln Ala Ala Glu Val Leu Ala Ser Leu Ala
595 600 605

Thr Arg Asp His Arg Glu Glu Val Glu Glu Asp Leu Ser Arg Met Leu
610 615 620

Ser Ala Gln Thr Gln Phe Gly Pro Thr Met Thr Gly Leu Pro Ser Gly
625 630 635 640

Leu Val Cys Thr Ala Gln Met Asn Cys Thr Ser His Leu Leu Thr Ile
645 650 655

Asn Asp Ile Leu Leu Gly His Gly Ala Ala Pro Val Gly Ser Met Gly
660 665 670

Cys Pro Leu Asp Phe Val Ser Tyr Gly Asp Asp Ile Val Leu Ala Met
675 680 685

Asp Asp Pro Lys Val Ala Thr Trp Leu Ile Asn Gly Trp Lys Lys Arg
690 695 700

Gly Phe Gln Ala Thr Ser Ala Ala Lys Thr Gly Pro Pro Gln Cys Gly
705 710 715 720

Lys Leu Gln Asp Met Thr Phe Ile Lys Arg Ser Phe Lys Lys Val Asp
725 730 735

Gly Glu Trp Arg Ala Pro Leu Glu Ala Ala Ser Ile Trp Lys Gly Leu
740 745 750

Ser Trp Met Arg Gly His Thr Ser Tyr Asp His Ser Glu Gly Val Gln
755 760 765

Asn Gly Asp Leu Thr Gly Thr Arg Ala Val Gly Val Phe Gln Ser Val
770 775 780

Met Ser Glu Phe Trp Gln His Gly Lys Glu Val Phe Glu Ala Ala Lys
785 790 795 800

Lys Leu Ile Ile Ser Tyr Ala Arg Glu Arg Lys Leu Arg Leu Pro Val
805 810 815

Ile Ile Pro Pro Tyr Ala Gln Phe Asn Pro Lys Glu Val Leu Ala Asp

820 825 830

Tyr Asn Arg Leu Ala Gly Glu Ser Ser Val Ser Met Leu Ser Val Ser
835 840 845

Trp His Thr Gly Gly Thr Gly Glu Val Gln Pro Val Ser Gln Thr Glu
850 855 860

Gly Pro Gly Gly Glu Asn Val Gly Glu Val Val Gly Leu Pro Ala Leu
865 870 875 880

Ala Thr Gly Leu Ala His Gln Thr Ala Gly Ile Glu Thr Ala Leu Ala
885 890 895

Thr Ser Gly Gly Pro Thr Val Gln Leu Asp Pro Ala Asn Arg Glu Arg
900 905 910

Phe Val Met Val Pro Gly Gly Met Val Ser Val Arg Thr Asn Met Thr
915 920 925

Arg Gly Ser Val Val Trp Arg Lys Lys Ile Ser Pro Gly Ile Asn Met
930 935 940

Trp Thr Asn Leu Leu Ser Ser Met Tyr Asn Ala Trp Cys Gly Gly Phe
945 950 955 960

Glu Val Met Ile Val Val Gly Ala Asn Asn Phe Ile Gly Gly Lys Phe
965 970 975

Leu Val Ala Tyr Phe Pro Pro Asn Val Ala Ser Gly Ser Tyr Ser Val
980 985 990

Glu Gln Val Thr Ala Phe Pro His Ala Ile Leu Asp Ile Arg Leu Met
995 1000 1005

Asp Asn Val Leu Leu Ala Cys Ser Asp Ile Lys Tyr Val Leu Trp
1010 1015 1020

His Pro Thr Asn Ala Ala Pro Glu Asp Pro Ile Ala Val Gly Gly
1025 1030 1035

Glu Val Val Val Tyr Leu Leu Thr Asn Ile Val Thr Ala Gly Asn
1040 1045 1050

Ser Gly Asn Val Leu Thr Leu Asp Met Ser Ile Phe Ser Arg Pro
1055 1060 1065

```
Met Pro  Asp Phe Asp Phe Asn  Phe Leu Met Pro Ile  Ser Leu Gly
    1070                 1075                 1080

Gly Ser  Gly Gly Pro Asn Asp  Leu Asp Met Gln Ala  Ala Gly Arg
    1085                 1090                 1095

Ala Leu  Cys Thr Pro Ala Thr  Ala Gly Gly Arg Gly  Trp His Val
    1100                 1105                 1110

Ile Asp  Val Leu Val Ala Thr  Gln Gln Ser Ser Thr  Val Ala Gly
    1115                 1120                 1125

Asp Met  Phe Ala Ser Thr Val  Arg Met Ser Gly Thr  Ser Pro Tyr
    1130                 1135                 1140

Cys Ile  Pro Tyr Pro Ala Ala  Asp Gly Met Ile Val  Glu Ala Ile
    1145                 1150                 1155

Lys Val  Ser Thr Thr Asp Tyr  Lys Leu Leu Cys Tyr  Thr Pro Asp
    1160                 1165                 1170

Gly Thr  Pro Trp Asp Val Gly  Thr Asp Lys Ala Ser  Lys Thr Leu
    1175                 1180                 1185

Pro Leu  Cys Trp Met Ala Tyr  Asn Ser Ala Ser Asp  His Thr Val
    1190                 1195                 1200

Leu Thr  Trp Tyr Lys Ser Asp  Gly Asn Leu Glu His  Thr Asn Gly
    1205                 1210                 1215

Lys Ile  Thr Leu Ala Ser Val  Gly Ala Gln Pro His  Tyr Val Pro
    1220                 1225                 1230

Tyr Ala  Ala Ala Glu Ile Pro  Ala Ala Thr Arg Ile  Ala Val Thr
    1235                 1240                 1245

Gly Ser  Lys Asp Gly Ala Ala  Leu Thr Asn Arg Glu  Ser Pro Ala
    1250                 1255                 1260

Ser Pro  Pro Ser Phe Thr Pro  Asn Asn Gly Glu Ser  Leu Ile Leu
    1265                 1270                 1275

Tyr Ala  Gly Leu Arg Lys Thr  Gly Gln Lys Gly Leu  Thr Leu Ser
    1280                 1285                 1290

Thr Gln  Glu Met Ile Thr Val  Phe Met Glu Ser Pro  Arg Val Thr
    1295                 1300                 1305
```

Ser Met Cys Gly Leu Phe Asn Val Ser Asp Ala Thr Gly Ala Thr
1310 1315 1320

Thr Val Gln Ala Lys Leu Tyr Pro Asn Gly Val Leu Thr Thr Gly
1325 1330 1335

Thr Ala Ala Ser Ala Ile Tyr Tyr Thr Gly Pro Ile Cys Phe Thr
1340 1345 1350

Tyr Val Gly Met Val Pro Val Asp Tyr Lys Leu Asn Pro Pro Ala
1355 1360 1365

Gly Gly Thr Asn Ala Asn Val Leu Asp Leu Val Glu Arg Leu Asp
1370 1375 1380

Lys Trp Leu Val Leu Gln Glu Arg Ser Gln Val Gly Ala Ser Ala
1385 1390 1395

Ser Ser Gln Ala Ser Leu Thr Gln Gln Leu Leu Ser Glu Ser Leu
1400 1405 1410

Gly Ser Ile Lys Arg Arg Pro Leu Lys Ser Lys Leu Glu Thr Leu
1415 1420 1425

Asp Ser Asp Glu Glu Ser Phe Glu Thr Ile Leu Gln Ala
1430 1435 1440

<210> 6
<211> 270
<212> PRT
<213> Salmonid calicivirus

<400> 6

Gln His Ala Ala Thr Phe Trp Arg Gly Val Asn Asn Pro Cys Asp Gly
1 5 10 15

Lys Cys Arg Ile Ser Leu Lys Asp Glu Leu Arg Pro Ala Lys Lys Arg
20 25 30

Glu Leu Gly Leu Thr Arg Pro Ile Phe Cys Phe Asp Val His Asp Thr
35 40 45

Val Arg Val Lys Ser Gln Ile Gly Ser Gly Leu Gln Ala Leu Ala Asp
50 55 60

Thr Cys Gly Thr His Ile Trp Ser Val Gly Ile Ser Gln Gln Asn Gly
65 70 75 80

Thr Trp Gly Gln Val Cys Glu Arg Leu Ser Lys Trp Arg Tyr Ala Met
85 90 95

Asp Ala Asp Phe Gly Arg Trp Asp Ser Thr Asn Ser His Pro Leu Met
100 105 110

Lys Gln Ala Ala Glu Val Leu Ala Ser Leu Ala Thr Arg Asp His Arg
115 120 125

Glu Glu Val Glu Glu Asp Leu Ser Arg Met Leu Ser Ala Gln Thr Gln
130 135 140

Phe Gly Pro Thr Met Thr Gly Leu Pro Ser Gly Leu Val Cys Thr Ala
145 150 155 160

Gln Met Asn Cys Thr Ser His Leu Leu Thr Ile Asn Asp Ile Leu Leu
165 170 175

Gly His Gly Ala Ala Pro Val Gly Ser Met Gly Cys Pro Leu Asp Phe
180 185 190

Val Ser Tyr Gly Asp Asp Ile Val Leu Ala Met Asp Asp Pro Lys Val
195 200 205

Ala Thr Trp Leu Ile Asn Gly Trp Lys Lys Arg Gly Phe Gln Ala Thr
210 215 220

Ser Ala Ala Lys Thr Gly Pro Pro Gln Cys Gly Lys Leu Gln Asp Met
225 230 235 240

Thr Phe Ile Lys Arg Ser Phe Lys Lys Val Asp Gly Glu Trp Arg Ala
245 250 255

Pro Leu Glu Ala Ala Ser Ile Trp Lys Gly Leu Ser Trp Met
260 265 270

<210> 7
<211> 521
<212> PRT
<213> Salmonid calicivirus

<400> 7

Val Ser Val Arg Thr Asn Met Thr Arg Gly Ser Val Val Trp Arg Lys
1 5 10 15

Lys Ile Ser Pro Gly Ile Asn Met Trp Thr Asn Leu Leu Ser Ser Met
20 25 30

```
Tyr Asn Ala Trp Cys Gly Gly Phe Glu Val Met Ile Val Val Gly Ala
        35                  40                  45

Asn Asn Phe Ile Gly Gly Lys Phe Leu Val Ala Tyr Phe Pro Pro Asn
    50                  55                  60

Val Ala Ser Gly Ser Tyr Ser Val Glu Gln Val Thr Ala Phe Pro His
65                  70                  75                  80

Ala Ile Leu Asp Ile Arg Leu Met Asp Asn Val Leu Leu Ala Cys Ser
                85                  90                  95

Asp Ile Lys Tyr Val Leu Trp His Pro Thr Asn Ala Ala Pro Glu Asp
            100                 105                 110

Pro Ile Ala Val Gly Gly Glu Val Val Val Tyr Leu Leu Thr Asn Ile
        115                 120                 125

Val Thr Ala Gly Asn Ser Gly Asn Val Leu Thr Leu Asp Met Ser Ile
    130                 135                 140

Phe Ser Arg Pro Met Pro Asp Phe Asp Phe Asn Phe Leu Met Pro Ile
145                 150                 155                 160

Ser Leu Gly Gly Ser Gly Gly Pro Asn Asp Leu Asp Met Gln Ala Ala
                165                 170                 175

Gly Arg Ala Leu Cys Thr Pro Ala Thr Ala Gly Gly Arg Gly Trp His
            180                 185                 190

Val Ile Asp Val Leu Val Ala Thr Gln Gln Ser Ser Thr Val Ala Gly
        195                 200                 205

Asp Met Phe Ala Ser Thr Val Arg Met Ser Gly Thr Ser Pro Tyr Cys
    210                 215                 220

Ile Pro Tyr Pro Ala Ala Asp Gly Met Ile Val Glu Ala Ile Lys Val
225                 230                 235                 240

Ser Thr Thr Asp Tyr Lys Leu Leu Cys Tyr Thr Pro Asp Gly Thr Pro
                245                 250                 255

Trp Asp Val Gly Thr Asp Lys Ala Ser Lys Thr Leu Pro Leu Cys Trp
            260                 265                 270

Met Ala Tyr Asn Ser Ala Ser Asp His Thr Val Leu Thr Trp Tyr Lys
        275                 280                 285
```

```
Ser Asp Gly Asn Leu Glu His Thr Asn Gly Lys Ile Thr Leu Ala Ser
    290                 295                 300

Val Gly Ala Gln Pro His Tyr Val Pro Tyr Ala Ala Ala Glu Ile Pro
305                 310                 315                 320

Ala Ala Thr Arg Ile Ala Val Thr Gly Ser Lys Asp Gly Ala Ala Leu
                325                 330                 335

Thr Asn Arg Glu Ser Pro Ala Ser Pro Pro Ser Phe Thr Pro Asn Asn
            340                 345                 350

Gly Glu Ser Leu Ile Leu Tyr Ala Gly Leu Arg Lys Thr Gly Gln Lys
        355                 360                 365

Gly Leu Thr Leu Ser Thr Gln Glu Met Ile Thr Val Phe Met Glu Ser
    370                 375                 380

Pro Arg Val Thr Ser Met Cys Gly Leu Phe Asn Val Ser Asp Ala Thr
385                 390                 395                 400

Gly Ala Thr Thr Val Gln Ala Lys Leu Tyr Pro Asn Gly Val Leu Thr
                405                 410                 415

Thr Gly Thr Ala Ala Ser Ala Ile Tyr Tyr Thr Gly Pro Ile Cys Phe
            420                 425                 430

Thr Tyr Val Gly Met Val Pro Val Asp Tyr Lys Leu Asn Pro Pro Ala
        435                 440                 445

Gly Gly Thr Asn Ala Asn Val Leu Asp Leu Val Glu Arg Leu Asp Lys
    450                 455                 460

Trp Leu Val Leu Gln Glu Arg Ser Gln Val Gly Ala Ser Ala Ser Ser
465                 470                 475                 480

Gln Ala Ser Leu Thr Gln Gln Leu Leu Ser Glu Ser Leu Gly Ser Ile
                485                 490                 495

Lys Arg Arg Pro Leu Lys Ser Lys Leu Glu Thr Leu Asp Ser Asp Glu
            500                 505                 510

Glu Ser Phe Glu Thr Ile Leu Gln Ala
        515                 520
```

<210> 8
<211> 171

<212> PRT
<213> Salmonid calicivirus

<400> 8

```
Glu Arg Phe Val Met Val Pro Gly Gly Met Val Ser Val Arg Thr Asn
1               5                   10                  15

Met Thr Arg Gly Ser Val Val Trp Arg Lys Lys Ile Ser Pro Gly Ile
            20                  25                  30

Asn Met Trp Thr Asn Leu Leu Ser Ser Met Tyr Asn Ala Trp Cys Gly
        35                  40                  45

Gly Phe Glu Val Met Ile Val Val Gly Ala Asn Asn Phe Ile Gly Gly
    50                  55                  60

Lys Phe Leu Val Ala Tyr Phe Pro Pro Asn Val Ala Ser Gly Ser Tyr
65                  70                  75                  80

Ser Val Glu Gln Val Thr Ala Phe Pro His Ala Ile Leu Asp Ile Arg
                85                  90                  95

Leu Met Asp Asn Val Leu Leu Ala Cys Ser Asp Ile Lys Tyr Val Leu
            100                 105                 110

Trp His Pro Thr Asn Ala Ala Pro Glu Asp Pro Ile Ala Val Gly Gly
        115                 120                 125

Glu Val Val Val Tyr Leu Leu Thr Asn Ile Val Thr Ala Gly Asn Ser
    130                 135                 140

Gly Asn Val Leu Thr Leu Asp Met Ser Ile Phe Ser Arg Pro Met Pro
145                 150                 155                 160

Asp Phe Asp Phe Asn Phe Leu Met Pro Ile Ser
                165                 170
```

<210> 9
<211> 360
<212> PRT
<213> Salmonid calicivirus

<400> 9

```
Leu Gly Gly Ser Gly Gly Pro Asn Asp Leu Asp Met Gln Ala Ala Gly
1               5                   10                  15

Arg Ala Leu Cys Thr Pro Ala Thr Ala Gly Gly Arg Gly Trp His Val
            20                  25                  30
```

```
Ile Asp Val Leu Val Ala Thr Gln Gln Ser Ser Thr Val Ala Gly Asp
        35                  40                  45

Met Phe Ala Ser Thr Val Arg Met Ser Gly Thr Ser Pro Tyr Cys Ile
    50                  55                  60

Pro Tyr Pro Ala Ala Asp Gly Met Ile Val Glu Ala Ile Lys Val Ser
65                  70                  75                  80

Thr Thr Asp Tyr Lys Leu Leu Cys Tyr Thr Pro Asp Gly Thr Pro Trp
                85                  90                  95

Asp Val Gly Thr Asp Lys Ala Ser Lys Thr Leu Pro Leu Cys Trp Met
            100                 105                 110

Ala Tyr Asn Ser Ala Ser Asp His Thr Val Leu Thr Trp Tyr Lys Ser
        115                 120                 125

Asp Gly Asn Leu Glu His Thr Asn Gly Lys Ile Thr Leu Ala Ser Val
    130                 135                 140

Gly Ala Gln Pro His Tyr Val Pro Tyr Ala Ala Ala Glu Ile Pro Ala
145                 150                 155                 160

Ala Thr Arg Ile Ala Val Thr Gly Ser Lys Asp Gly Ala Ala Leu Thr
                165                 170                 175

Asn Arg Glu Ser Pro Ala Ser Pro Pro Ser Phe Thr Pro Asn Asn Gly
            180                 185                 190

Glu Ser Leu Ile Leu Tyr Ala Gly Leu Arg Lys Thr Gly Gln Lys Gly
        195                 200                 205

Leu Thr Leu Ser Thr Gln Glu Met Ile Thr Val Phe Met Glu Ser Pro
    210                 215                 220

Arg Val Thr Ser Met Cys Gly Leu Phe Asn Val Ser Asp Ala Thr Gly
225                 230                 235                 240

Ala Thr Thr Val Gln Ala Lys Leu Tyr Pro Asn Gly Val Leu Thr Thr
                245                 250                 255

Gly Thr Ala Ala Ser Ala Ile Tyr Tyr Thr Gly Pro Ile Cys Phe Thr
            260                 265                 270

Tyr Val Gly Met Val Pro Val Asp Tyr Lys Leu Asn Pro Pro Ala Gly
```

```
                275                 280                 285

        Gly Thr Asn Ala Asn Val Leu Asp Leu Val Glu Arg Leu Asp Lys Trp
            290                 295                 300

        Leu Val Leu Gln Glu Arg Ser Gln Val Gly Ala Ser Ala Ser Ser Gln
        305                 310                 315                 320

        Ala Ser Leu Thr Gln Gln Leu Leu Ser Glu Ser Leu Gly Ser Ile Lys
                        325                 330                 335

        Arg Arg Pro Leu Lys Ser Lys Leu Glu Thr Leu Asp Ser Asp Glu Glu
                    340                 345                 350

        Ser Phe Glu Thr Ile Leu Gln Ala
                355                 360
```

<210> 10
<211> 20
<212> DNA
<213> Artificial sequence

<220>
<223> PCR primer

<400> 10
gtacaccacc ctggttgagg 20

<210> 11
<211> 20
<212> DNA
<213> Artificial sequence

<220>
<223> PCR primer

<400> 11
cagcgacagc cttctgaact 20

<210> 12
<211> 20
<212> DNA
<213> Artificial sequence

<220>
<223> PCR primer

<400> 12
agggattccc cattgttagg 20

<210> 13
<211> 18
<212> DNA
<213> Artificial sequence

<220>
<223> PCR primer

<400> 13
gcagcaagga tggagctg 18

<210> 14
<211> 20
<212> DNA
<213> Artificial sequence

<220>
<223> PCR primer

<400> 14
agttcaaccc gaaggaggtt 20

<210> 15
<211> 20
<212> DNA
<213> Artificial sequence

<220>
<223> PCR primer

<400> 15
tggtaggcca acgacttctc 20

<210> 16
<211> 20
<212> DNA
<213> Artificial sequence

<220>
<223> PCR primer

<400> 16
ccataggcca agaaaccagg 20

<210> 17
<211> 20
<212> DNA
<213> Artificial sequence

<220>
<223> PCR primer

<400> 17
gcctttgaag tccaagctcg 20

<210> 18
<211> 20
<212> DNA
<213> Artificial sequence

<220>
<223> PCR primer

<400> 18
gttcctggtg gcctacttcc 20

<210> 19
<211> 21
<212> DNA
<213> Artificial sequence

<220>
<223> PCR primer

<400> 19
acattgccac tgttgccagc c 21

## Description

### Field of invention

The present invention pertains to a novel ethiological agent, a virus identified in Atlantic salmon. The invention further relates to subject-matter which is useful for controlling viral infections, including the isolated virus, immunological compositions and diagnostic tools.

### Background of the invention

Caliciviridae are small, non-enveloped, positive-stranded RNA viruses. The Caliciviridae are at present divided into five groups, tentatively designated distinct genera, on the basis of sequence relatedness and genomic organization (2). Four groups are known human pathogens, while the fifth group Lagovirus, where Rabbit Haemmoraghic Virus is a member, is not known to be a human pathogen. The only group of caliciviruses which can be grown *in vitro,* is the group of marine caliciviruses belonging to the genus Vesivirus, where the virus causing vesicular exanthema of swine (VES) is a member. The virus causing vesicular exanthema of swine (VES) has been known to have a reservoir in marine mammals, and can also infect swine (1). Many species of marine mammals are known to be susceptible to calicivirus infection. A calicivirus has also been found in a marine fish species, the opaleye perch (3), and this virus belonged to the same group, Vesivirus, as the VES-virus found in marine mammals.

Caliciviruses are believed to be involved in disease development in salmonids. The disease is systemic and can result in inflammation of the heart. The liver and kidney tissue can also be affected, as well as other tissues. While infection with calicivirus in salmonids may in many cases be asymptomatic, the presence of calicivirus in fish for human consumption is nevertheless highly undesirable. Consequently there is an urgent need to isolate and characterize the calicivirus which infect salmonids and to devise prophylactic and therapeutic approaches to the control of such infections.

### Summary of the invention

An object of the present invention relates to an isolated non-enveloped, positive stranded RNA virus, wherein said virus is selected from the group consisting of:

I) the viral isolate A2-G01 deposited under the Budapest Treaty with the European Collection of Cell culture (ECACC), Health Protection Agency, Porton Down, Salisbury, Wiltshire (UK), SP4 0JG UK on January 7 2010 under deposit number 10010701; and

II) a virus which comprises in its genome a ribonucleic acid sequence which by reverse transcription and 2$^{nd}$ strand synthesis provides a sequence which is at least 65 % identical to the sequence set forth in SEQ ID NO: 1, and/or is at least 65 % identical to the sequence set forth in SEQ ID NO: 2.

Further objects of the invention relate to isolated nucleic acid sequences, isolated or recombinant proteins or amino acid sequences, antibodies, immunogenic compositions, diagnostic methods and kits and the virus, isolated nucleic acid sequences and isolated or recombinant amino acid sequences for use in medicine.

### Description of the drawings

Figure 1: Schematic representation of polyprotein RHDVgp1 and parts of processed products: RHase-dependent RNA polymerase (RdRp) and coat1 (major coat protein).

Figure 2: GF-1 cells infected with 33% cell culture medium from cells displaying cpe. 17 days after infection (A) and GF-1 control cells, not infected, 17 days after infection (B).

Figure 3. Amino acid sequence from open reading frame (ORF) in the genome of salmonid calicivirus isolate A2-G01: Sequence marked in bold: RdRp (specific domain hit), underlined sequence: Coat (specific domain hit) comprising putative S-domain, underlined sequence marked in bold: Coat protein, putative P-domain.

Figure 4. Alignment of coat protein from different caliciviruses.

Figure 5. Phylogram obtained by Maximum likelihood (ML) analysis in the software package MEGA 5 of the conserved region in the putative capsid protein of selected Caliciviruses. A model for amino acid substitution (rtREV+G+F) was selected using Model Selection. The tree was bootstrapped with 100 replicates under a ML criterion and midpoint rooted for presentation. The bootstrap consensus tree is shown.

Figure 6. Phylogram obtained by Maximum likelihood (ML) analysis in the software package MEGA 5 of the conserved region in the putative RNA dependent RNA polymerase of selected Caliciviruses. A model for amino acid substitution (WAG+G+I) was selected using Model Selection. The tree was bootstrapped with 100 replicates under a ML criterion and midpoint rooted for presentation. The bootstrap consensus tree is shown.

Figure 7. Phylogram obtained by Maximum likelihood (ML) analysis in the software package MEGA 5 of the putative protease domain of selected Caliciviruses. A model for amino acid substitution (WAG+G) was selected using Model Selection. The tree was bootstrapped with 100 replicates under a ML criterion and midpoint rooted for presentation. The bootstrap consensus tree is shown.

Figure 8. Phylogram obtained by Maximum likelihood (ML) analysis in the software package MEGA 5 of the putative RNA Helicase domain of selected Caliciviruses. A model for amino acid substitution (WAG+G+I) was selected using Model Selection. The tree was bootstrapped with 100 replicates under a ML criterion and midpoint rooted for presentation. The bootstrap consensus tree is shown.

Figure 9. Receipt for deposition, viral isolate A2-G01.

**Detailed description of the invention**

The present invention provides a new calicivirus isolated from Atlantic salmon in Norway. The new calicivirus shares only limited sequence homology with the other known caliciviruses, and will probably constitute a new genus under the species calicivirus. The present inventors propose the name salmonid calicivirus.

The virus was isolated by seeding of homogenate from diseased fish onto cell culture. The material was passaged several times through cell culture before a cytopathogenic effect was observed. The genome was cloned and sequenced using a subtraction cloning method. The isolated virus and the nucleic acid sequences and amino acid sequences according to the present invention allows preparation of vaccines and diagnostic tools for calicivirus infections in fish.

Definitions

The term "isolated" when applied in relation to a nucleic acid sequence or amino acid sequence, refers to the nucleic acid sequence or amino acid sequence in a preparation containing reduced amounts of the material with which the nucleic acid sequence or amino acid sequence is natively associated.

In particular, the term "isolated" may refer to a "substantially pure nucleic acid sequence" or a "substantially pure amino acid sequence" meaning a preparation of the nucleic acid sequence or the amino acid sequence which contains at most 5% by weight of other nucleic acid sequences and/or amino acid sequences with which it is natively associated (lower percentages of other nucleic acid sequences and/or amino acid sequences are preferred, e.g. at most 4%, at most 3%, at most 2%, at most 1%, and at most ½%). It is preferred that the substantially pure nucleic acid sequence is at least 96% pure, *i.e.* that the nucleic acid sequence or a plasmid or vector comprising the nucleic acid sequence constitutes at least 96% by weight of the total nucleic acid sequences present in the preparation, and higher percentages are preferred, such as at least 97%, at least 98%, at least 99%, at least 99,25%, at least 99,5%, and at least 99,75%. Likewise it is preferred that the amino acid sequence constitutes at least 96% by weight of the total amino acid sequences present in the preparation, and higher percentages are preferred, such as at least 97%, at least 98%, at least 99%, at least 99,25%, at least 99,5%, and at least 99,75%. This definition does not exclude the situation where a substantially pure amino acid sequence is combined with other substantially pure amino acid sequences, e.g. in a composition for therapeutic use.

It is especially preferred that the nucleic acid sequence or amino acid sequence is in "essentially pure form", *i.e.* that the nucleic acid sequence or amino acid sequence is free of any other nucleic acid sequences and/or amino acid sequences with which it is natively associated, *e.g.* free of any other nucleic acid sequences and/or amino acid sequences natively found in cells from piscine species, in particular salmonid species. This can be accomplished by preparing the nucleic acid sequence or amino acid sequence by means of recombinant methods in a non-piscine host cell, such as a non-salmonid host cell, as will be described in detail below, or by synthesizing the amino acid sequence by the well-known methods of solid or liquid phase peptide synthesis.

When used in relation to the virus of the present invention, the term "isolated" refers to the virus when separated from its natural environment, such as the cells and tissues from its piscine host. In particular, the term "isolated" refers to a culture, such as a pure culture of the virus derived from a tissue sample from an infected host. As viruses cannot reproduce on their own and use host cell machinery to make both the viral genome and capsids, the term "isolated" may in particular refer to a culture of cells infected with the virus.

The term "isolated" may further refer to a virus which is substantially free of other viral or microbial material. The term "substantially free of other viral or microbial material" as used herein refers to a preparation of a virus in which other viral or microbial material cannot be detected using conventional techniques like seeding on TSA or cystein heart agar spread plates, seeding in cell cultures known to support the growth of fish viruses like Pancreas Disease Virus, Infectious Salmon Anemia virus and Infectious pancreatic Necrosis virus and PCR with primers designed against known sequences from fish pathogens. Further, it is to be understood that when "substantially free of other viral or microbial material" the virus of the invention is in a form wherein it may be used for therapeutic purposes. This definition does not exclude applications in which a pure culture of the virus according to the invention is combined with other vaccine antigens, e.g. other bacterial or viral antigens in compositions, e.g. compositions intended for vaccination purposes.

The term "sequences identity" indicates a quantitative measure of the degree of homology between two amino acid sequences or between two nucleic acid sequences. If the two sequences to be compared are not of equal length, they must be aligned to give the best possible fit, allowing the insertion of gaps or, alternatively, truncation at the ends of the polypeptide sequences or nucleotide sequences. The sequence identity can be calculated as $\frac{(N_{ref}-N_{dif})100}{N_{ref}}$, wherein Ndif is the total number of non-identical residues in the two sequences when aligned and wherein Nref is the number of residues in one of the sequences. Hence, the DNA sequence AGTCAGTC will have a sequence identity of 75% with the sequence AATCAATC (Ndif=2 and Nref=8). A gap is counted as non-identity of the specific residue(s), i.e. the DNA sequence AGTGTC will have a sequence identity of 75% with the DNA sequence AGTCAGTC (Ndif=2 and Nref=8).

With respect all embodiments of the invention relating to nucleotide sequences, the percentage of sequence identity between one or more sequences may also be based on alignments using the clustalW software (http:/www.ebi.ac.uk/clustalW/index.html) with default settings. For nucleotide sequence alignments these settings are: Alignment=3Dfull, Gap Open 10.00, Gap Ext. 0.20, Gap separation Dist. 4, DNA weight matrix: identity (IUB). For amino acid sequence alignments the settings are as follows: Alignment=3Dfull, Gap Open 10.00, Gap Ext. 0.20, Gap separation Dist. 4, Protein weight matrix: Gonnet.

Alternatively, nucleotide sequences may be analysed using programme DNASIS Max and the comparison of the sequences may be done at http://www.paralign.org/. This service is based on the two comparison algorithms called Smith-Waterman (SW) and ParAlign. The first algorithm was published by Smith and Waterman (1981) and is a well established method that finds the optimal local alignment of two sequences. The other algorithm, ParAlign, is a heuristic method for sequence alignment; details on the method are published in Rognes (2001). Default settings for score matrix and Gap penalties as well as E-values were used.

The term "immunogenic." when used in relation to a polypeptide or protein or in relation to a subsequence of a polypeptide or protein implicates the ability of said polypeptide, protein or subsequence to elicit an immune response in a biological sample or in an individual currently or previously infected with a salmonid calicivirus, such as a salmonid calicivirus as defined hereinbelow.

The immune response to a polypeptide, a protein or a subsequence of a polypeptide or protein may in particular be a humoral response as determined by a specific antibody response in an immune or infected individual. The presence of antibodies in serum may be determined *in vitro* by the ELISA technique or in a Western blot where the polypeptide or protein or the subsequence of a polypeptide or protein is absorbed to either a nitrocellulose membrane or a polystyrene surface. By the use of labeled secondary antibodies the presence of specific antibodies can be determined by measuring the optical density (O.D.) e.g. by Enzyme-linked immunosorbent assay (ELISA) where a positive response is a response of more than background plus two standard deviations or alternatively a visual response in a Western blot.

The serum may preferably be diluted in PBS from 1:10 to 1:100 and added to the absorbed polypeptide, protein or subsequence of polypeptide or protein. The serum is preferably incubated with the absorbed polypeptide, protein or subsequence of polypeptide or protein from 1 to 12 hours.

Another relevant approach to determining an immune response to a polypeptide, a protein or a subsequence of a polypeptide or protein is measuring the protection in animal models induced after vaccination with the polypeptide, the protein or the subsequence of a polypeptide or protein in an adjuvant. A suitable animal model in this context is a salmonid. Read-out for induced protection could be decrease of the bacterial load in target organs compared to non-vaccinated animals, prolonged survival times compared to non-vaccinated animals and diminished weight loss compared to non-vaccinated animals.

The term "immunogenic." when used in relation to a polypeptide or protein or in relation to a subsequence of a polypeptide or protein may further imply the presence within said polypeptide, protein or said subsequence of a polypeptide or protein of one or more immunogenic portions, such as one or more epitopes for B-cells and/or one or more epitopes for T-cells. B-cell epitopes, in particular, can be determined by analysing the B cell recognition to overlapping peptides covering the polypeptide of interest.

As the skilled person will understand, "cytopathogenic effect" refers to visible morphologic changes in cells infected with viruses. It may in particular include shutdown of cellular RNA and protein synthesis, cell fusion, release of lysosomal enzymes, changes in cell membrane permeability, diffuse changes in intracellular structures, presence of viral inclusion bodies, and chromosomal aberrations.

"Cell culture" refers to cultures of cells, such as transformed cells, established *in vitro.* Specifically, as used herein, "cell line" refers to a population of cells capable of continuous or prolonged growth and division *in vitro.* Often, cell lines are clonal populations derived from a single progenitor cell. It is further known in the art that spontaneous or induced changes can occur in karyotype during storage or transfer of such clonal populations. Therefore, cells derived from the cell line referred to may not be precisely identical to the ancestral cells or cultures, and the cell line referred to includes such variants. The term "cell lines" also includes immortalized cells.

Finally, in the context of the present invention, a "polyvalent vaccine" (also referred to as a "multivalent vaccine") is used to define a combination of several antigens in one vaccine. Thus, a polyvalent vaccine may protect against more than one disease. As opposed to a polyvalent vaccine, a "monovalent vaccine" is a vaccine containing vaccine components directed at only one pathogen. In particular, a monovalent vaccine may contain only one antigen, protecting against one particular disease.

In a first aspect the invention provides an isolated non-enveloped, positive stranded RNA virus, wherein said virus is selected from the group consisting of:

I) the viral isolate A2-G01 deposited under the Budapest Treaty with the European Collection of Cell culture (ECACC), Health Protection Agency, Porton Down, Salisbury, Wiltshire (UK), SP4 0JG UK on January 7 2010 under deposit number 10010701; and

II)a virus which comprises in its genome a ribonucleic acid sequence which by reverse transcription and 2nd strand synthesis provides a sequence which is at least 65% identical to the sequence set forth in SEQ ID NO: 1 and/or is at least 65% identical to the sequence set forth in SEQ ID NO: 2.

Alternatively phrased, the said virus II) comprises in its genome a ribonucleic acid sequence, which is at least 65% identical to the sequence set forth in SEQ ID NO: 3 and/or is at least 65% identical to the sequence set forth in SEQ ID NO: 4.

In further embodiments the virus comprises, in its genome, a nucleic acid sequence which by reverse transcription and 2nd strand synthesis provides a sequence which is at least 70% identical, such as at least, 75%, 80%, 85%, 90%, 95%, 96%, 97%, 98%, 99%, 99.5%, 99.8% or 99.9% identical, to the sequence set forth in SEQ ID NO: 1 and/or to the sequence set forth in SEQ ID NO: 2. According to these embodiments the virus comprises, in its genome, a nucleic acid sequence which is at least 70% identical, such as at least, 75%, 80%, 85%, 90%, 95%, 96%, 97%, 98%, 99%, 99.5%, 99.8% or 99.9% identical, to the sequence set forth in SEQ ID NO: 3 and/or to the sequence set forth in SEQ ID NO: 4.

According to specific embodiments of the invention, the virus comprises in its genome a ribonucleic acid sequence which is 100% identical to the sequence set forth in SEQ ID NO: 3 and/or is 100% identical to the sequences set forth in SEQ ID NO: 4 so that said sequence by reverse transcription and 2nd strand synthesis provides the DNA sequence set forth in SEQ ID NO: 1 and/or SEQ ID NO: 2.

In particular embodiments the virus may comprise a sequence which has been derived from the sequence set forth in SEQ ID NO: 3 and/or from the sequence set forth in SEQ ID NO: 4 by deletion, addition and/or modification of one or more ribonucleic acid residues, such as from 1-10 ribonucleic acid residues, such as from 11-20 ribonucleic acid residues, from 21-30 ribonucleic acid residues or from 31-50 ribonucleic acid residues. In particular 1, 2, 3, 4, 5, 6, 7, 8, 9, 10, 11, 12, 13, 14, 15, 16, 17, 18, 19 or 20 ribonucleic acid residues may have been deleted, modified or added. According to these embodiments, reverse transcription and 2nd strand synthesis results in a sequence that differs from the sequence set forth in SEQ ID NO: 1 and/or from the sequence set forth in SEQ ID NO: 2 by deletion, addition and/or modification of one or more nucleic acid residues, such as from 1-10 nucleic acid residues, such as from 11-20 nucleic acid residues, from 21-30 nucleic acid residues or from 31-50 nucleic acid residues. In particular the sequence differs from the sequence set forth in SEQ ID NO: 1 and/or from the sequence set forth in SEQ ID NO: 2 by deletion, addition and/or modification of 1, 2, 3, 4, 5, 6, 7, 8, 9, 10, 11, 12, 13, 14, 15, 16, 17, 18, 19 or 20 nucleic acid residues.

Studies of the genomic sequence of a salmonid calicivirus according to the present invention has identified one open reading frame. The open reading frame comprises nucleic acid residues 113-4438 in the sequence set forth in SEQ ID NO: 1. This ORF encodes a polyprotein, RHDVgp1, which is processed into at least an RNA-dependent RNA polymerase (RdRp) and coat1 (major coat protein). The polyprotein and the processed products are shown in Figure 1.

The virus according to the invention may further be a virus wherein the genome of said virus comprises open reading frame encoding a sequence selected from the group consisting of:
I) The amino acid sequence set forth in any one of SEQ ID NO: 5, SEQ ID NO: 6, SEQ ID NO: 7, SEQ ID NO: 8 and/or SEQ ID NO: 9.
II) An amino acid sequence which is at least 75% identical to any one of the sequences in I), such as an amino acid sequence which has been derived from any of said sequences in I) and/or II) by deletion, addition and/or modification of one or more amino acid residues.

Table 1: Nucleotide and amino acid sequences according to the invention.

| | |
|---|---|
| SEQ ID NO: 1 | Nucleotide sequence from the genome of salmonid calicivirus isolate A2-G01 (nucleic acid residues 1- 4759) |
| SEQ ID NO: 2 | Nucleotide sequence of the full length genome of salmonid calicivirus isolate A2-G01 (nucleic acid residues 1- 7433) |
| SEQ ID NO: 3 | Ribonucleotide sequence from the genome of salmonid calicivirus isolate A2-G01 (nucleic acid residues 1- 4759) |
| SEQ ID NO: 4 | Ribonucleotide sequence of the full length genome of salmonid calicivirus isolate A2-G01 (nucleic acid residues 1- 7433) |
| SEQ ID NO: 5 | Amino acid sequence from open reading frame (ORF) in the genome of salmonid calicivirus isolate A2-G01 |
| SEQ ID NO: 6 | Partial amino acid sequence of the RNA-dependent RNA polymerase deduced from the open reading frame of salmonid calicivirus isolate A2-G01 (residues 485-755 of SEQ ID NO: 5) |
| SEQ ID NO: 7 | SEQ ID NO: 7. Amino acid sequence of coat protein deduced from the open reading frame of salmonid |
| | calicivirus isolate A2-G01, including putative S- and P-domains (residues 920-1441 of SEQ ID NO: 5) |
| SEQ ID NO: 8 | Partial amino acid sequence of coat protein deduced from the open reading frame of salmonid calicivirus isolate A2-G01 (residues 911-1081 of SEQ ID NO: 5) |
| SEQ ID NO: 9 | Sequence from the putative P-domain of salmonid calicivirus isolate A2-G01 (residues 1081-1441 of SEQ ID NO: 5) |

In particular, the genome of said virus may comprise an open reading frame encoding a sequence which is at least 80%, 85%, 90%, 95%, 96%, 97%, 98%, 99%, 99.5%, 99.8% or 99.9% identical to the amino acid sequence set forth in any of SEQ ID NO: 5, SEQ ID NO: 6, SEQ ID NO: 7, SEQ ID NO: 8 or SEQ ID NO: 9. In particular, the virus may comprise a sequence which has been derived from any of SEQ ID NO: 5, SEQ ID NO: 6, SEQ ID NO: 7, SEQ ID NO: 8 or SEQ ID NO: 9 by deletion, addition and/or modification of one or more amino acid residues, such as from 1-10 amino acid residues, such as from 11-20 amino acid residues, from 21-30 amino acid residues or from 31-50 amino acid residues. In particular 1, 2, 3, 4, 5, 6, 7, 8, 9 or 10 amino acid residues may have been deleted, modified or added.

In further embodiments the genome of said virus encodes an RNA-dependent RNA polymerase comprising an amino acid sequence selected from the group consisting of

I) The amino acid sequence set forth in SEQ ID NO: 6;
II) An amino acid sequence which is at least 75% identical, such as at least 80%, 85%, 90%, 95%, 96%, 97%, 98%, 99%, 99.5%, 99.8% or 99.9% identical, to the sequence in I), such as an amino acid sequence which has been derived from said sequence in I) by deletion, addition and/or modification of one or more amino acid residues, such as from 1-10 amino acid residues, such as from 11-20 amino acid residues, from 21-30 amino acid residues or from 31-50 amino acid residues. In particular 1, 2, 3, 4, 5, 6, 7, 8, 9 or 10 amino acid residues may have been deleted, modified or added.

In even further embodiments the genome of said virus encodes a coat protein comprising an amino acid sequence selected from the group consisting of

I) The amino acid sequence set forth in SEQ ID NO: 7, SEQ ID NO: 8 and/or SEQ ID NO: 9;

II) An amino acid sequence which is at least 75% identical, such as at least 80%, 85%, 90%, 95%, 96%, 97%, 98%, 99%, 99.5%, 99.8% or 99.9% identical, to the sequence in I), such as an amino acid sequence which has been derived from said sequence in I) by deletion, addition and/or modification of one or more amino acid residues, such as from 1-10 amino acid residues, such as from 11-20 amino acid residues, from 21-30 amino acid residues or from 31-50 amino acid residues. In particular 1, 2, 3, 4, 5, 6, 7, 8, 9 or 10 amino acid residues may have been deleted, modified or added.

In particular, the virus according the invention may be an attenuated or inactivated virus.

As the skilled person will realize, replication of RNA virus genomes is accompanied by very high mutation rates (Watson et al. 1987). This is due to the lack of proofreading activity of RNA virus polymerases, which leads to a constant generation of new genetic variants e.g. during virus propagation (Elena and Sanjuán (2005). The virus according to the invention may therefore, in certain embodiments, be a virus, which is obtainable by growing viral isolate A2-G01 on a cell culture, such as for one or more passages, such as from 2-20 passages, from 5-20 passages, from 10-20 passages, from 2-15 passages, from 2-10 passages, from 2-5 passages, from 5-20 passages, from 5-15 passages, from 5-10 passages or such as for 2, 3, 4, 5, 6, 7, 8, 9, 10, 11, 12, 13, 14, 15, 16, 17, 18, 19 or 20 passages, and/or until a cytopathogenic effect is obtained. Preferably, such a cell culture is selected from the group consisting of: GF-1 cells (derived from the fin tissue of a grouper (Epinephelus coioides), CHH-1 cells (available from ATCC under deposit number CRL-1680), CHSE-214 cells (available from ATCC, deposit number CRL 1681 and from ECACC under deposit number 91041114). In each passage, viral infection of said cells may be obtained by infection with 1/3 of the culture medium from the previous passage + 2/3 of fresh medium.

Viral isolates, which are within the scope of the present invention, may be identified as being capable of binding to an antibody, which is raised against viral isolate A2-G01 as deposited under deposit number 10010701 and/or being capable of binding to an antibody, which is raised against an amino acid sequence as set forth in any of SEQ ID NOs: 5-9. The said antibody may be a polyclonal antibody (antiserum), such as an antiserum from a salmonid which has been infected with viral isolate A2-G01, or it may be a monoclonal antibody. It will be within the capacity of a person of skills in the art to produce such antibodies, using conventional technology available in the art.

According to other aspects the invention provides an isolated nucleic acid sequence encoding an amino acid sequence as defined above.

In particular embodiments the invention pertains to a nucleic acid sequence, which comprises or consists of a sequence selected from the group consisting of:

I) The nucleic acid sequence set forth in SEQ ID NO: 1 and/or SEQ ID NO: 2;

II) A subsequence of the nucleic acid sequence set forth in SEQ ID NO: 1 or in SEQ ID NO: 2, wherein the subsequence has a length of from 20-7400 consecutive nucleic acid residues; and

III) A nucleic acid sequence which is at least 75 % identical, such as at least 80%, 85%, 90%, 95%, 96%, 97%, 98%, 99%, 99.5%, 99.8% or 99.9% identical, to any one of the sequences in I) or II), such as a sequence which has been derived from the sequence set forth in SEQ ID NO: 1 or 2 by deletion, addition and/or modification of one or more nucleic acid residues, such as from 1-10 nucleic acid residues, such as from 11-20 nucleic acid residues, from 21-30 nucleic acid residues or from 31-50 nucleic acid residues. In particular the sequence may differ from the sequence set forth in SEQ ID NO: 1 or 2 by deletion, addition and/or modification of 1, 2, 3, 4, 5, 6, 7, 8, 9, 10, 11, 12, 13, 14, 15, 16, 17, 18, 19 or 20 nucleic acid residues,

According to particular embodiments the said subsequence of the nucleic acid sequence set forth in SEQ ID NO: 1 or SEQ ID NO: 2 has a length of from 20-50, 20-100, 20-150, 20-200, 30-50, 30-100, 30-150, 30-200, 40-100, 40-150, 40-200, 50-100, 50-150, 50-200, 50 -300, 50-400, 50-500, 50-750, 50-1500, 50-2000, 50-3000, 50-4000, 50-4500, 50-4700, 100-150, 100-200, 100-500, 100-1000, 100-2000, 100-3000, 100-4000, 100-4500, 100-4700, 200-1000, 200-3000, 200-4000, 200-4500, 200-4700, 500-1000, 500-2000, 500-3000, 500-4000, 500-4500, 500-4700, 1000-2000, 1000-3000, 1000-4000, 1000-4500, 1000-4700, 2000-3000, 2000-4000, 2000-4500, 2000-4700, 3000-3500, 3000-4000, 3000-4500, 3000-4700, 3500-4000, 3500-4500, 3500-4700, 3000-4750, 4000-4500, 4000-4700, 4000-4750, 4500-4700 or from 4000-4750 consecutive nucleic acid residues.

In further embodiments the said nucleic acid sequence and/or subsequence is a sequence which encodes an amino acid sequence, which is immunogenic.

In further embodiments the said subsequence in II) is selected from the group consisting of:

i) a sequence comprising nucleic acid residues 1568-2377 of the sequence set forth in SEQ ID NO: 1 or SEQ ID NO: 3; corresponding to nucleic acid residues 4241-5051 of the sequence set forth in SEQ ID NO: 2 or SEQ ID NO: 4 (sequence encoding SEQ ID NO: 6); and

ii) a sequence comprising nucleic acid residues 2842-3355 of the sequence set forth in SEQ ID NO: 1 or SEQ ID NO: 3; corresponding to nucleic acid residues 5517-6029 of the sequence set forth in SEQ ID NO: 2 or SEQ ID NO: 4 (sequence encoding SEQ ID NO: 8).

In particularly preferred embodiments the said subsequence in II) is selected from the group consisting of:

i) A sequence comprising nucleic acid residues 2872-4435 of the sequence set forth in SEQ ID NO: 1 or SEQ ID NO: 3; corresponding to nucleic acid residues 4546-7109 of the sequence set forth in SEQ ID NO: 2 or SEQ ID NO: 4 (sequence encoding SEQ ID NO: 7); and
ii) A sequence comprising nucleic acid residues 3356-4435 of the sequence set forth in SEQ ID NO: 1 or SEQ ID NO: 3; corresponding to nucleic acid residues 5547-7109 of the sequence set forth in SEQ ID NO: 2 or SEQ ID NO: 4 (sequence encoding SEQ ID NO: 9);
iii) a sequence comprising nucleic acid residues 3356-3862 of the sequence set forth in SEQ ID NO: 1 or SEQ ID NO: 3; corresponding to nucleic acid residues 5547-6536 of the sequence set forth in SEQ ID NO: 2 or SEQ ID NO: 4 (sequence encoding amino acid residues 1081-1250 of SEQ ID NO: 5);
iv) a sequence comprising nucleic acid residues 3637-4435 of the sequence set forth in SEQ ID NO: 1 or SEQ ID NO: 3; corresponding to nucleic acid residues 6311-7109 of the sequence set forth in SEQ ID NO: 2 or SEQ ID NO: 4 (sequence encoding amino acid residues 1175-1441 of SEQ ID NO: 5).

A further aspect of the invention provides a vector comprising a nucleic acid sequence as defined above.
Yet a further aspect of the invention relates to a host cell comprising the isolated nucleic acid sequence defined above; and/or the vector defined above.
Other aspects of the invention provide an isolated or recombinant amino acid sequence or an isolated or recombinant protein comprising or consisting of one or more amino acid sequences selected from the group consisting of

I) The amino acid sequence set forth in SEQ ID NO: 5 or an amino acid sequence contained within SEQ ID NO:5, selected from the group consisting of the amino acid sequences set forth in any of SEQ ID NOs: 6-9;
II) A subsequence of any one of the amino acid sequences set forth in any of SEQ ID NO: 5-7, which has a length of 30-200 consecutive amino acid residues, or a subsequence of any one of the amino acid sequences set forth in either of SEQ ID NO: 8 or SEQ ID NO: 9, which has a length of 30-170 consecutive amino acid residues;
III) An amino acid sequence which is at least 75% identical, such as at least 80%, 85%, 90%, 95%, 96%, 97%, 98%, 99%, 99.5%, 99.8% or 99.9% identical, to any of the sequences in I) or II), such as an amino acid sequence which has been derived from any of said sequences in I) or II) by deletion, addition and/or modification of one or more amino acid residues, such as from 1-10 amino acid residues, such as from 11-20 amino acid residues, from 21-30 amino acid residues or from 31-50 amino acid residues. In particular 1, 2, 3, 4, 5, 6, 7, 8, 9 or 10 amino acid residues may have been deleted, modified or added.

The skilled person will expect that considerable alterations may be introduced into the amino acid sequences defined in SEQ ID NOs: 5-9 and subsequences thereof without significantly altering their overall structure, function and immunological properties. In particular the skilled person will expect that an amino acid sequence will have the same immunological properties as a sequence defined in SEQ ID NO: 5, 6, 7, 8 or 9 or a subsequence thereof, as long as it is at least 75% identical to the defined sequence. This is supported for instance by Cothia et al. EMBO j., vol 5, 4, pp. 823-826, 1986, who investigated the relationship between the divergence of sequence and structure in proteins. Cothia concluded: "A protein structure will provide a close general model for other proteins with which its sequence homology is >50%. If the homology drops to 20% there will be large structural differences that are at present impossible to predict."
The said subsequence of the amino acid sequence set forth in SEQ ID NO: 5 may have a length of from 30-50, 30-100, 30-150, 30-200, 40-100, 40-150, 40-200, 50-100, 50-150, 50-200, 100-150, 100-200 consecutive amino acid residues. The said subsequences of the amino acid sequence set forth in SEQ ID NO: 6 may have a length of from 30-50, 30-100, 30-150, 30-200, 40-100, 40-150, 40-200, 50-100, 50-150, 50-200, 100-150, 100-200 consecutive amino acid residues. Likewise the said subsequence of the amino acid sequence set forth in SEQ ID NO: 7 may have a length of from 30-50, 30-100, 30-150, 30-160, 30-170, 40-100, 40-150, 40-160, 40-170, 50-100, 50-150, 50-160, 50-170, 100-150, 100-160 100-170, 100-180, 100-200 consecutive amino acid residues. Further, the said subsequence of the amino acid sequence set forth in SEQ ID NO: 8 may have a length of from 30-50, 30-100, 30-150, 30-160, 30-170, 40-100, 40-150, 40-160, 40-170, 50-100, 50-150, 50-160, 50-170, 100-125, 100-150 or 100-160 consecutive amino acid residues. Finally, the said subsequence of the amino acid sequence set forth in SEQ ID NO: 9 may have a length of from 30-50, 30-100, 30-150, 30-160, 30-170, 40-100, 40-150, 40-160, 40-170, 50-100, 50-150, 50-160, 50-170, 100-125, 100-150 or 100-160 consecutive amino acid residues.
In particular embodiments the said isolated or recombinant amino acid sequence and/or the said isolated or recombinant protein and/or the said subsequences are immunogenic. The invention in particular relates to a subsequence of the amino acid sequences set forth in SEQ ID NOs: 5-9, wherein said subsequence is immunogenic.

Particularly preferred subsequences according to the present invention include a subsequence of the putative P-domain of coat protein comprising amino acid residues 1081-1250 of SEQ ID NO: 5, and a subsequence of the putative P-domain of coat protein comprising amino acid residues 1175-1441 of SEQ ID NO: 5.

The invention also provides an antibody, which is raised against an amino acid sequence as defined above. It is to be understood that the antibody may be a monoclonal or polyclonal antibody.

The invention also relates to an isolated nucleic acid sequence which is complementary to and/or hybridizes under high stringency conditions to a nucleic acid sequence as defined above. In particular the sequence may be complementary to and/or hybridizes under high stringency conditions to a nucleic acid sequence being selected from the group consisting of:

I) The nucleic acid sequence set forth in SEQ ID NO: 1 or SEQ ID NO: 2;

II) A subsequence of the nucleic acid sequence set forth in SEQ ID NO: 1 or SEQ ID NO: 2, wherein the subsequence has a length of from 20-7400 consecutive nucleic acid residues; and

III) A nucleic acid sequence which is at least 75 % identical, such as at least 80%, 85%, 90%, 95%, 96%, 97%, 98%, 99%, 99.5%, 99.8% or 99.9% identical, to any one of the sequences in I) or II)

As the skilled person will appreciate, high stringency conditions may comprise hybridization in a buffer consisting of 6 X SSC, 50 mM Tris-HCL (pH=7.5), 1 mM EDTA, 0.02% PVP, 0.02% Ficoll, 0.02% BSA and 100 μg/ml denatured salmon sperm DNA, for 48 hours at 65°C, washing in a buffer consisting of 2 X SSC, 0.01% PVP, 0.01% Ficoll, 0.01% BSA for 45 minutes at 37°C, and washing in a buffer consisting of 0.1 X SSC, for 45 minutes at 50°C.

According to the invention is also provided an immunogenic composition/vaccine comprising:

I) An isolated virus as defined above;

II) An isolated or recombinant amino acid sequence/an isolated or recombinant protein as defined above;

III) An isolated nucleic acid sequence as defined above; or

IV) A vector according to the invention.

The immunogenic composition/vaccine preferably comprises a virus as defined above, and a pharmaceutically acceptable carrier and/or excipient.

The skilled person will acknowledge that, when contemplated for the purpose of eliciting a protective immune response, the composition according to the invention may further comprise an organic adjuvant and/or an inorganic adjuvant.

The organic adjuvant is preferably selected from the group consisting of: mineral oil, squalene (2,6,10,15,19,23-hexamethyl-2,6,10,14,18,22-tetracosahexaene), virosomes, CpG oligodeoxynucleotides and adjuvants based on pharmaceutical oils and purified surfactants, such as the adjuvants sold under the trade name Montanide™. Other examples of adjuvants frequently used in fish and shellfish farming are muramyldipeptides, lipopolysaccharides, several glucans and glycans, mineral oil and Carbopol®. Also adjuvants such as interleukin and glycoproteins may be used. An extensive overview of adjuvants suitable for fish and shellfish vaccines is given in the review paper by Jan Raa (1996).

Useful inorganic adjuvants will be known to the skilled artisan and are described in JC Aguilar and EG Rodriguez, 2007, Vaccine 25, 3752 - 3762.

In particular, the inorganic adjuvant which is optionally included in the composition according to the invention is selected from the group consisting of $Al(OH)_3$ (Aluminium hydroxide), $Ca_3(PO4)_2$ (Calcium phosphate), and water unsoluble salts of aluminium, calcium, iron or zirconium.

The vaccine may also comprise a so-called "vehicle". A vehicle is a device to which the antigen adheres, without being covalently bound to it. Such vehicles are i.a. biodegradable nano/micro-particles or capsules of PLGA (poly-lactide-*co*-glycolic acid), alginate or chitosan, liposomes, niosomes, micelles, multiple emulsions and macrosols, all known in the art. A special form of such a vehicle, in which the antigen is partially embedded in the vehicle, is the so-called ISCOM (European patents EP 109.942, EP 180.564 and EP 242.380).

In addition, the composition may comprise one or more suitable surface-active compounds, detergents and/or emulsifiers.

In particular, the detergent is selected from the group consisting of non-ionic detergents, cationic detergents and anionic detergents.

As the skilled person will realize esters of non-PEG-ylated or PEG-ylated sorbitan with fatty acids are examples of useful emulsifiers. The emulsifier may in particular be polysorbate or sorbitan oleate. More specifically, the said detergent and/or emulsifier may be selected from the group consisting of polyoxyethylene (20) sorbitan monooleate (Tween®80), Sorbitane monooleate (Span®80), Cremophore, Tween® and Span®.

Preferably, the immunogenic composition/vaccine contains a virus according to the invention wherein said virus is either attenuated or inactivated.

As the skilled person will know the virus may be inactivated using several procedures known in the art, such as by chemical or physical means. Chemical inactivation may for instance be carried out by treatment of the virus with enzymes, with formaldehyde, β-propiolactone or ethyleneimine or a derivative thereof, with organic solvent (e.g. 30 halogenated hydrocarbon) and/or detergent, e.g. Triton® or Tween®. Physiological inactivation can advantageously be carried out by subjecting the viruses to energy-rich radiation, such as UV light, gamma irradiation or X-rays. For the present purpose, the immunogenic composition/vaccine preferably contains virus according to the invention, wherein said virus has been inactivated by treatment with formalin.

Preferably, the immunogenic composition/vaccine contains virus, wherein said virus is formulated in a water-in-oil emulsion.

In preferred embodiments the immunogenic composition is a recombinant vaccine comprising one or more amino acid sequences according to the invention.

In particular preferred embodiments the recombinant vaccine comprises one or more amino acid sequence selected from the group consisting of:

I) The amino acid sequence set forth in SEQ ID NO: 5 or an amino acid sequence contained within SEQ ID NO:5, selected from the group consisting of the amino acid sequences set forth in any of SEQ ID NOs:6-9;
II) A subsequence of any of the amino acid sequences set forth in any of SEQ ID NO: 5-7, which has a length of 30-200 consecutive amino acid residues, or a subsequence of any one of the amino acid sequences set forth in either of SEQ ID NO: 8 or SEQ ID NO: 9, which has a length of 30-170 consecutive amino acid residues;
III) An amino acid sequence which is at least 75% identical to any one of the sequences in I) or II), such as an amino acid sequence which has been derived from any of said sequences in I) or II) by deletion, addition and/or modification of one or more amino acid residues.

In particular embodiments the said isolated or recombinant amino acid sequence and/or the said isolated or recombinant protein and/or the said subsequences are immunogenic. The invention in particular relates to vaccine wherein the subsequence of the amino acid sequences set forth in any of SEQ ID NOs: 5-9 is immunogenic.

Particularly preferred vaccines according to the present invention comprise a subsequence of the putative P-domain of the coat protein comprising amino acid residues 1081-1250 of SEQ ID NO: 5, and/or a subsequence of the putative P-domain of the coat protein (comprising amino acid residues 1175-1441 of SEQ ID NO: 5).

According to further embodiments, the vaccine comprises a subsequences of the amino acid in I) in combination with an amino acid sequences as defined in III).

In still further embodiments, the vaccine comprises multiple subsequences of the amino acid in I) and/or multiple amino acid sequences as defined in III).

Such recombinant vaccines may be prepared using standard procedures known in the art.

According to certain embodiments the immunogenic composition/vaccine comprises virus or one or more isolated or recombinant amino acid sequences or proteins according to the invention wherein said virus or said isolated or recombinant amino acid sequence(s) or proteins is/are combined with other immunologic agents. The skilled person will realize the potential advantage of such polyvalent vaccines.

The invention further provides a method of detecting a virus as described above in a sample, comprising contacting said sample with a nucleic acid sequence; or with an antibody as described above.

A further aspect of the invention provides a diagnostic kit comprising a nucleic acid sequence as described above; or an antibody according to the invention.

The invention also relates to a method of manufacturing an immunogenic composition or vaccine as described above, said method comprising:

I) Growing a virus as defined above in a cell culture, such as until a cytopathogenic effect is observed
II) Harvesting the virus; and
III) Optionally inactivating the virus.

According to further aspects the invention provide a virus as defined above, an isolated nucleic acid sequence, or an isolated or recombinant amino acid sequence/an isolated or recombinant protein as defined above for use in medicine. In particular, said virus, said isolated nucleic acid sequence, and said isolated or recombinant amino acid sequence/isolated or recombinant protein may be for use in preventing infections with salmonid calicivirus or reducing the incidence of such infections, or for treatment of such infections.

Further aspects of the invention provide the use of a virus, an isolated nucleic acid sequence, or an isolated or recombinant amino acid sequence/an isolated or recombinant protein as defined above for the manufacture of an immunological composition for preventing infections with salmonid calicivirus or reducing the incidence of such infections, or for treatment of such infections.

Even further aspects of the disclosure pertain to a method for preventing, treating or reducing the incidence of infections with salmonid calicivirus comprising identifying an individual which is infected or is at risk of becoming infected with said virus; and administering to said individual virus, an isolated nucleic acid sequence, or an isolated or recombinant amino acid sequence/an isolated or recombinant protein as defined above.

It should be noted that embodiments and features described in the context of one of the aspects of the present invention also apply to the other aspects of the invention.

The invention will now be described in further details in the following non-limiting examples.

## Examples

Example 1: Isolation of salmonid calicivirus

GF-1 cell cultures were grown in L-15 (Sigma) with 10% foetal bovine serum (FBS, Gibco), 1% glutamine (Sigma) and 0,1% gentamicin (Sigma). GF-1 cells were seeded at a density of 3,5E4 GF-1 cells per $cm^2$ and infected with heart homogenates from farmed Atlantic salmon in Norway experiencing undiagnosed disease with heart pathology.

The homogenate was prepared by homogenizing heart tissue with ca 5 ml cell culture medium and quartz sand with a pistil in a mortar. The homogenate was centrifuged at 1000 x g for 5 minutes in a centrifuge, the supernatant removed and passed through a 0.45 μm filter. 0.1 ml filtered supernatant was added to a 75 $cm^2$ cell culture flask. After 28 days of incubation at 15°C, a mild cytopathogenic effect was observed with the cell layer displaying reduced growth and some rounding up of cells. 0.5 ml supernatant from this culture was transferred to a new 75$cm^2$ cell flask with GF-1 cells, and the same type of cpe was observed 28 days after inoculation. New naïve GF-1 cells were infected with 1/3 inoculate from these flasks + 2/3 of fresh medium, and a stronger cpe of the same type was observed.

Images of GF-1 cells infected with 33% cell culture medium from cells displaying cpe. 17 days after infection and GF-1 control cells, not infected, are shown in figure 2(A) and (B), respectively.

Example 2: Cloning and sequencing of a nucleotide sequence from the genome of salmonid calicivirus isolate A2-G01

Total RNA was isolated from calicivirus-infected GF-1 cells showing CPE or PD-infected GF-1 cells using Trizol LS according to manufacturers protocol. All RNAs were DNase treated using Turbo DNA-*free*™ kit (Ambion), followed by routine DNase treatment using 10 μg RNA in a 50 μl reaction. RNA integrity of all RNA isolations (no degradation products) were verified by Bioanalyzer analysis. All measurements of RNA concentrations were made by Picodrop spectrophotometry (Picodrop Limited, Saffron Walden, UK).

96 different hexamer primers that had been shown to rarely prime rRNA but did prime all known mammalian viruses (D. Endoh, et.al., 2005, Nucleic Acid Research, Vol 33, No.6) were ordered and mixed to a concentration of 0.36 ug/ul.

Double-stranded (ds) cDNA was synthesized using "Superscript double-stranded cDNA synthesis kit" from Invitrogen, applying 1ul of 0.36 ug/ul "non-ribosomal" random hexamer primers for first strand synthesis. 1 μg of totalRNA was used in first strand synthesis.

Representational difference analysis (RDA)

RDA was performed according to a modified protocol by Pastorian, K. et.al. (Analytical Biochemistry 283, 89-98 (2000)). Both forward and reverse RDA were performed, one of which PD-infected GF-1 cells served as the driver, and one of which the calicivirus infected GF-1 cells served as the driver.

*Driver* cDNA was made accordingly:

> Concentrations of dsCDNAs were measured before the dscDNAs were digested by DPN II and purified. Purified restriction cut dscDNA were subsequently ligated to 24mer I, and amplified by PCR. An optimal number of amplification cycles were identified for each cDNA to avoid "over-amplification", and thus that just a few cDNAs "take over" the PCR reactions. Amplified cDNAs were subsequently purified, digested, and purified again before concentrations were measured.

*Tester* cDNA for hybridization round I was made by ligation of 0.2 ug of driver cDNA to RDA 24mer II.

Hybridization round I was performed according to Pastorian, K. et.al., at a ratio of 1:500 tester:driver. After hybridization, two optimalized rounds of PCR were performed according to Pastorian, K. et.al., before purification, DPN II digestion, and purification again. Subtracted, purified, restriction cut amplified tester from Hybridization I was then subjected to two more rounds of hybridization at the ratios 1:5000 (hyb II), and 1: 50000 (hyb III), by ligation to adapters/primers 24mer III and 24mer IV.

All PCR reactions were visualized by gel electrophoresis. Resulting cDNAs (after three rounds of hybridizations) were transformed into Topo TA vectors from Invitrogen and sequenced.

Several clones were obtained from the RDA experiment. The sequences were BLASTed and primers were designed for unknown sequences. The primers were used to screen the clones for sequences that were present in cells having been infected with the virus, but not in uninfected cells. The primers that were found to produce a PCR product from virus cultures (primers for clones A2, A8, C3, E12 and G01, see figure 1) were then combined to find the chronology of the sequences. Primers E12-forward together with C3-forward (E12fC3f) produced a band of about 1.5 kb. Likewise, A2f-E12r produced a band of 2,5-3 kb, and G01f-C3r produced a product of 600 bp. No match was found for the A8 clone. The obtained PCR-products were all cloned and sequenced. The sequences of the A2, E12, C3 and G01 clones were retrieved in the products of these primer combinations, serving as a good control for the specificity of the PCR (i.e the A2 clone sequence was found in the 5'-end and the E12 sequence was found in the 3'end of the A2fE12r PCR products and so forth). The PCR analysis and the sequencing suggested that the clones were in the order A2, E12, C3, G01. To test the robustness of this approach a PCR reaction with relevant primer combinations (G01fA2f, G01f, E12f, G01fC3r, A2fE12r and A2fC3f) that would amplify long products were set up. The longest band produced was E12fG01f. The lack of a A2-G01 band was most likely due to technical problems. The E12fG01f PCR product was cloned and sequenced and found to contain the C3 clone and confirmed that the E12-C3-G01 sequence was correct. Now, a continuous sequence of 4472 bp (SEQ ID NO: 1) had been identified. By DNA analysis software (pDRAW, Acaclone), one long open reading frame was found. By running BLAST on the sequence, putative regions were identified, such as a domain with Peptidase homology, a domain with calicivirus RdRp homology and a domain with virus coat homology. On the basis of this sequence, one primer pair in the RdRp region, and two primer pairs in the coat region were constructed. These primer pairs were used to screen field samples for the presence of this virus. The virus sequences were indeed found in field samples. To identify the remaining sequences of the virus, 3'RACE and 5'RACE was performed. The 3' RACE ready cDNA was produced using a oligo(dT)-primer with a 22 bp tag. The 3'RACE PCR product was produced using the G01r primer and a primer recognizing the tag. A product of about 400 bp was obtained, cloned and sequenced. The sequence contained the G01 clone sequence followed by new 3' sequence and a poly(A) tail. The final virus sequence after 3'RACE is 4759 bp (SEQ ID NO: 2).

Table 2: Primer sequences

| Primer | Forward primer | Reverse primer |
|---|---|---|
| A2 | GTACACCACCCTGGTTGAGG (SEQ ID NO: 10) | CAGCGACAGCCTTCTGAACT (SEQ ID NO: 11) |
| C3 | AGGGATTCCCCATTGTTAGG (SEQ ID NO: 12) | GCAGCAAGGATGGAGCTG (SEQ ID NO: 13) |
| E12 | AGTTCAACCCGAAGGAGGTT (SEQ ID NO: 14) | TGGTAGGCCAACGACTTCTC (SEQ ID NO: 15) |
| G01 | CCATAGGCCAAGAAACCAGG (SEQ ID NO: 16) | GCCTTTGAAGTCCAAGCTCG (SEQ ID NO: 17) |

Example 3: Detection of salmonid calicivirus in Atlantic salmon by Real Time PCR

Tissue samples from heart were collected from a clinical trial and from cages in a commercial salmon farm.

1. Clinical trial: In the clinical trial, fish were injected with salmonid calicivirus grown as described in example 1. The fish were held in salt water, 12°C. Sampling was done before challenge, and 1,4,8 and 11 weeks after challenge of the fish. The tip of the heart ventricle and mid kidney was removed aseptically, and transferred to RNAlater (Ambion). Following RNA isolation with standard procedures, total RNA (800ng) was reverse transcribed into cDNA and real-time PCR was performed, both using SuperScript™ III Platinum® Two-Step qRT-PCR Kit with SYBR® Green (Invitrogen) according to the manufacturer's instructions. The reaction conditions were UDG-incubation at 50°C for 2 minutes, activation of the hot-start polymerase at 95°C for 2 minutes, followed by 40-45 cycles of 95°C for 15 seconds, primer annealing for 15 seconds and extension for 1 min at 60 °C. Melting curve analysis was performed to confirm formation of expected PCR products, and results are presented as Ct-values. PCR primers were A2 forward: GTACACCACCCTGGTTGAGG and A2 reverse: CAGCGACAGCCTTCTGAACT

| Fish nr | weeks post challenge | Challenge | Organ | Heart | Kidney |
|---|---|---|---|---|---|
| 1 | 0 | Injected | Heart | Negative | Negative |
| 2 | | | | Negative | Negative |
| 3 | | | | Negative | Negative |
| 4 | | | | Negative | Negative |

(continued)

| Fish nr | weeks post challenge | Challenge | Organ | Heart | Kidney |
|---|---|---|---|---|---|
| 25 | 1 | Injected | Heart | 30,92 | 29,31 |
| 26 | | | | 28,2 | 28,22 |
| 27 | | | | 29,52 | 30,15 |
| 28 | | | | 39,01 | 28,65 |
| 49 | 4 | Injected | Heart | 29,11 | 28,82 |
| 50 | | | | 28,05 | 27,65 |
| 51 | | | | 30,74 | 28,79 |
| 52 | | | | 28,74 | 28,76 |
| 73 | 8 | Injected | Heart | 32,58 | 28,15 |
| 74 | | | | 31,65 | 28,35 |
| 75 | | | | 30,94 | 27,97 |
| 76 | | | | 32,18 | 29,32 |
| 121 | 11 | Injected | Heart | Negative | 29,37 |
| 122 | | | | 32,69 | 27,81 |
| 123 | | | | 30,31 | 25,77 |
| 124 | | | | 33,69 | 28,72 |

These result show that Real Time RT-PCR is a suitable method for detection of salmonid calicivirus in tissue samples from fish.

2. Field samples: Heart samples were collected on RNAlater from 20 farms experiencing disease problems related to unspecific clinical signs and heart pathology. Following RNA isolation with standard procedures, total RNA (800ng) was reverse transcribed into cDNA and real-time PCR was performed, both using SuperScript™ III Platinum® Two-Step qRT-PCR Kit with SYBR® Green (Invitrogen) according to the manufacturer's instructions. The reaction conditions were UDG-incubation at 50°C for 2 minutes, activation of the hot-start polymerase at 95°C for 2 minutes, followed by 40-45 cycles of 95°C for 15 seconds, primer annealing for 15 seconds and extension for 1 min at 60°C. Melting curve analysis was performed to confirm formation of expected PCR products, and results are presented as Ct-values. PCR primers were forward: GTTCCTGGTGGCCTACTTCC (SEQ ID NO: 19) and A2 reverse: ACATTGCCACTGTTGCCAGCC (SEQ ID NO: 20). Of the 20 farms tested, 5 were positive for salmon calicivirus.

Example 4. Phylogenetic analysis

The nucleotide sequence of salmonid calicivirus was subjected to bioinformatic analysis to infer its relationship to other caliciviruses. First, the amino acid sequence of the polyprotein was translated from the identified ORF using the Expasy translate tool. Conserved domains showing significant identity to RNA helicase, protease, RNA dependent RNA polymerase and calicivirus capsid proteins were identified in the polyprotein by Blast analysis.

Phylogenetic analysis was performed on the individual domains that were identified in the polyprotein by Blast analysis, the RNA helicase, the protease, the RNA dependent RNA polymerase and the capsid. In these analyses, the alignment was cropped to contain only the conserved domains identified by the initial Blast analysis. The model of substitution was chosen independently for each analysis using Model Selection with the Bayes Information Criterion in MEGA. In the phylogenetic analysis, the salmon calicivirus is named PHARMAQ calicivirus.

The phylogenetic trees are shown in figures 5-9.

Example 5: Vaccination with an inactivated virus from isolate A2-G01.

Preparation of vaccine: Salmonid calicivirus was grown as described in example 1 in a 632 $cm^2$ flask with 30000 GF-1 cells/$cm^2$, 155 ml medium and 25 ml inoculate prepared as described in example 1. Supernatant was harvested 28 days after inoculation, formalin added to 2% and incubated at 15°C for 72 hours. Inactivated virus was pelleted by centrifugation at 100,000 xg for 4 hours, and resuspended in 6 ml PBS. 5ml of this was formulated in a total of 50 ml water-in-oil emulsion.

Fish were vaccinated with a 0.1 ml dose of vaccine. 500 degree days after vaccination, the fish were challenged intraperitoneally with virus grown as described in example 1. Sampling was done before challenge, and 1,8 and 11 weeks after challenge of the fish. The tip of the heart ventricle and mid kidney was removed aseptically, and transferred to RNAlater (Ambion). Following RNA isolation with standard procedures, total RNA (800ng) was reverse transcribed into cDNA and real-time PCR was performed, both using SuperScript™ III Platinum® Two-Step qRT-PCR Kit with SYBR® Green (Invitrogen) according to the manufacturer's instructions. The reaction conditions were UDG-incubation at 50°C for 2 minutes, activation of the hot-start polymerase at 95°C for 2 minutes, followed by 40-45 cycles of 95°C for 15 seconds, primer annealing for 15 seconds and extension for 1 min at 60°C. Melting curve analysis was performed to confirm formation of expected PCR products, and results are presented as Ct-values. PCR primers were A2 forward: GTACACCACCCTGGTTGAGG and A2 reverse: CAGCGACAGCCTTCTGAACT

| Fish nr | WPC | | Heart | Kidney | Average |
|---|---|---|---|---|---|
| 1 | 0 | Unvaccinated | Negative | Negative | |
| 2 | | | Negative | Negative | |
| 3 | | | Negative | Negative | |
| 4 | | | Negative | Negative | |
| 73 | 8 | Unvaccinated | 32,58 | 28,15 | 28,4 |
| 74 | | | 31,65 | 28,35 | |
| 75 | | | 30,94 | 27,97 | |
| 76 | | | 32,18 | 29,32 | |
| 121 | 11 | Unvaccinated | Negative | 29,37 | 27,9 |
| 122 | | | 32,69 | 27,81 | |
| 123 | | | 30,31 | 25,77 | |
| 124 | | | 33,69 | 28,72 | |
| 97 | 8 | Vaccinated | Negative | 32,54 | 32,3 |
| 98 | | | Negative | Negative | |
| 99 | | | Negative | 31,03 | |
| 100 | | | Negative | 33,22 | |
| 145 | 11 | Vaccinated | Negative | 32,42 | 32,0 |
| 146 | | | Negative | 31,99 | |
| 147 | | | Negative | 31,87 | |
| 148 | | | Negative | 31,8 | |

In the non-vaccinated controls, salmonid calicivirus was detected in both heart and kidney at all sampling time points. In the vaccinated group, virus was only detected in the kidney, and with higher Ct values than in the control group. The detected virus in the kidney may be originating from the vaccine, or may be a low amount of virus having replicated in the kidney. This experiment shows that the vaccine with inactivated virus has protected the fish against a challenge with salmonid calicivirus.

Example 6: Vaccination with an inactivated variant of viral isolate A2-GO1.

Virus from isolate A2-G01 are cultured in serial passages on GF-1 cells, in a total of 12 passages.

For preparation of the vaccine the virus is grown, harvested, inactivated and formulated in a water-in-oil emulsion as described in example 5.

Fish are vaccinated, subsequently challenged intraperitoneally with virus. Detection of virus infection is performed essentially as described in example 5. Example 7: Identification of immunogenic regions within the amino acid sequence of the coat protein of salmonid calicivirus:

The amino acid of the coat domain from salmon calicivirus isolate A2-G01 was aligned with coat domains from other calici viruses, including Southampton virus, Norwalk virus, Feline calicivirus, Rabbit hemorrhagic disease virus, Porcine enteric sapovirus, Calicivirus NB, Newbury agent 1, Sapporo virus, European brown hare syndrome virus, San Miguel sea lion virus, Human calicivirus, Murine norovirus 1, Tulane virus and Feline calicivirus.

By the alignment several conserved residues were identified. The conserved residues are expected to be important features of the coat protein, and are found within part of the S-domain which is the best conserved part of the coat protein. Identification of the conserved residues in the S-domain by alignment is shown in Figure 4.

A review of the literature on calicivirus has revealed that, generally, the majority of the neutralizing epitopes are located in the P-domain, Hence, the P-domain is expected to be the best choice for a recombinant protein vaccine. The putative P-domain of salmon calicivirus is set forth in SEQ ID NO: 9.

Example 8: Preparation of antibodies/antisera

Preparation of monoclonal antibodies

BALBc mice are immunized with purified salmon calicivirus. Using Indirect Fluorescent Antibody test (IFA) with calicivirus-infected GF-1 cells, antiviral antibodies are evaluated in sera collected 15 days after the second immunization. Seropositive mice, which show a high titre of antibodies against salmon calicivirus, are inoculated with the purified salmon calicivirus by the intravenous route as the third immunization. Subsequently, spleen cells of the mice are taken out and fused with myeloma cells. Hybridomas secreting antibodies against calicivirus are detected by IFA and a neutralization test against calicivirus. Cultures producing virus-specific antibodies are subcloned by limiting dilution, followed by recloning and preparation of ascetic fluid.

Preparation of antisera against coat protein

A nucleic acid sequence encoding the putative coat protein is cloned into an expression vector and is subsequently expressed in a protein expression system. The polypeptide is purified and injected into mice or rabbits. Sera obtained from the animals are inactivated and stored at -20°C until used.

Example 9: Preparation of salmon calicivirus recombinant vaccines

For the purpose of vaccination the following recombinant polypeptides are provided:

1) Coat protein, comprising amino acid residues residues 920-1441 of SEQ ID NO: 5
2) Sequence from the putative P-domain of coat protein (comprising amino acid residues 1081-1441 of SEQ ID NO: 5)
3) Sequence from the putative S-domain of coat protein (comprising amino acid residues 911 - 1081 of SEQ ID NO: 5)
4) Subsequence of the putative P-domain of coat protein (comprising amino acid residues 1081-1250 of SEQ ID NO: 5)
5) Subsequence of the putative P-domain of coat protein (comprising amino acid residues 1175-1441 of SEQ ID NO: 5)
6) Subsequence of the coat protein (comprising amino acid residues 990-1375 of SEQ ID NO: 5)

The polypeptides are cloned into expression vectors and expressed in a protein expression system. Each polypeptide is purified, and formulated as a water-in-oil emulsion.

Example 10: Vaccination and challenge of Atlantic salmon with salmon calicivirus

The aim of this study is to assess the effect of vaccines based on recombinant coat proteins from salmon calicivirus.

Atlantic salmon are vaccinated by intraperitoneal vaccination with the vaccines described in example 9. Vaccine efficacy is tested through i.p. challenge of vaccinated and unvaccinated Atlantic salmon. Tissue samples from vaccinated and unvaccinated fish will be collected for histopathological evaluation and PCR-screening.

Mortality is registered daily and tissue samples are collected at different time points from the vaccinated and unvaccinated group for histopathological evaluation and PCR-screening.

**References**

1. Smith AW, Boyt PM J Zoo Wildlife Med 1990;21: 3-23

2. Berke et al., J Med Virol1997 Aug 52(4) 419-424

3. Smith et al. Diseases of Aquatic organisms 1986;2:73-80

4. Rognes T (2001), ParAlign: a parallel sequence alignment algorithm for rapid and sensitive database searches, Nucleic Acids Research, 29, 1647-1652 2001.

5. Smith TF and Waterman MS (1981) Journal of Molecular Biology, 147, 195-197.

6. Watson JD, Hopkins NH, Roberts JW, Steitz JA, Weiner AM (1987) Molecular biology of the gene, Chapter 24 The extraordinary diversity of eucaryotic viruses (898-961).

7. Elena, SF and Sanjuán, R et al. (2005), Journal of Virology, 79, 11555-11558.

8. Pastorian, K. et al. (2000) Analytical Biochemistry 283, 89-98.

9. Endoh, D. et al., (2005), Nucleic Acid Research, Vol 33, No.6.

10. Raa, J. (1996), Reviews in Fisheries Science 4(3): 229-228.

11. Aguilar, JC and Rodriguez, EG, (2007), Vaccine 25, 3752 - 3762.

12. Cothia et al. (1986) EMBO j., vol 5, 4, pp. 823-826.

## Claims

1. An isolated non-enveloped, positive stranded RNA virus, wherein said virus is selected from the group consisting of:

   I) the viral isolate A2-G01 deposited under the Budapest Treaty with the European Collection of Cell culture (ECACC), Health Protection Agency, Porton Down, Salisbury, Wiltshire (UK), SP4 OJG UK on January 7 2010 under deposit number 10010701; and
   II) a virus which comprises in its genome a ribonucleic acid sequence which by reverse transcription and $2^{nd}$ strand synthesis provides a sequence which is at least 65 % identical to the sequence set forth in SEQ ID NO: 1, and/or is at least 65 % identical to the sequence set forth in SEQ ID NO: 2.

2. The virus according to claim 1, wherein the genome of said virus comprises an open reading frame encoding a sequence selected from the group consisting of

   I) The amino acid sequence set forth in any of SEQ ID NO: 5, SEQ ID NO: 6, SEQ ID NO: 7, SEQ ID NO: 8 and/or SEQ ID NO: 9
   II) An amino acid sequence which is at least 75% identical to any of the sequences in I).

3. The virus according to claim 1 or 2, wherein the genome of said virus encodes an RNA-dependent RNA polymerase comprising an amino acid sequence selected from the group consisting of

   I) The amino acid sequence set forth in SEQ ID NO: 6;
   II) An amino acid sequence which is at least 75% identical to the sequence in I).

4. The virus according to claim 1 or 2, wherein the genome of said virus encodes a coat protein comprising an amino acid sequence selected from the group consisting of

   I) The amino acid sequence set forth in SEQ ID NO: 8, SEQ ID NO: 7 and/or SEQ ID NO: 9;
   II) An amino acid sequence which is at least 75% identical to the sequence in I).

5. The virus according to any of the preceding claims, said virus being an attenuated or inactivated virus.

6. The virus according to any of the preceding claims, wherein said virus is obtainable by growing viral isolate A2-G01 as deposited under deposit number 10010701 on a cell culture, such as for one or more passages and/or until a cytopathogenic effect is obtained.

7. The virus according to any of the preceding claims, said virus being capable of binding to an antibody which is raised against viral isolate A2-G01 as deposited under deposit number 10010701 and/or being capable of binding to an antibody which is raised against the amino acid sequence as set forth in any of SEQ ID NOs: 5-9, wherein the antibody is a polyclonal antibody (antiserum), or a monoclonal antibody.

8. An isolated nucleic acid sequence encoding an amino acid sequence selected from the group consisting of

I) The amino acid sequence set forth in any of SEQ ID NO: 5, SEQ ID NO: 6, SEQ ID NO: 7, SEQ ID NO: 8 and/or SEQ ID NO: 9
II) An amino acid sequence which is at least 75% identical to any of the sequences in I).

**9.** The isolated nucleic acid sequence according to claim 8, said nucleic acid sequence comprising or consisting of a sequence selected from the group consisting of:

I) The nucleic acid sequence set forth in SEQ ID NO: 1 and/or SEQ ID NO: 2;
II) A subsequence of the nucleic acid sequence set forth in SEQ ID NO: 1 or in SEQ ID NO: 2, wherein the subsequence has a length of from 20-7400 consecutive nucleic acid residues; and
III) A nucleic acid sequence which is at least 75% identical to any one of the sequences in I) or II).

**10.** The isolated nucleic acid sequence according to claim 9 in which said subsequence in II) is selected from the group consisting of:

i) a sequence comprising nucleic acid residues 1568-2377 of the sequence set forth in SEQ ID NO : 1
ii) a sequence comprising nucleic acid residues 2842-3355 of the sequence set forth in SEQ ID NO: 1
iii) a sequence comprising nucleic acid residues 2872-4435 of the sequence set forth in SEQ ID NO: 1
iv) a sequence comprising nucleic acid residues 3356-4435 of the sequence set forth in SEQ ID NO: 1
v) a sequence comprising nucleic acid residues 3356-3862 of the sequence set forth in SEQ ID NO: 1 and
vi) a sequence comprising nucleic acid residues 3637-4435 of the sequence set forth in SEQ ID NO : 1.

**11.** Vector comprising a nucleic acid sequence according to any of claims 8 to 10.

**12.** A host cell comprising the isolated nucleic acid sequence according to any of claims 8 to 10; and/or the vector according to claim 11.

**13.** An isolated or recombinant amino acid sequence/an isolated or recombinant protein comprising or consisting of one or more sequences selected from the group consisting of

I) The amino acid sequence set forth in any of SEQ ID NO: 5-9;
II) A subsequence of any one of the amino acid sequences set forth in any of SEQ ID NO: 5-7, which has a length of 30-200 consecutive amino acid residues, or a subsequence of any one of the amino acid sequences set forth in either of SEQ ID NO: 8 or SEQ ID NO: 9, which has a length of 30-170 consecutive amino acid residues;
III) An amino acid sequence which is at least 75% identical to the sequences in I) or II).

**14.** An antibody, which is raised against an amino acid sequence selected from the group consisting of

I) The amino acid sequence set forth in any of SEQ ID NO: 5, SEQ ID NO: 6, SEQ ID NO: 7, SEQ ID NO: 8 and/or SEQ ID NO: 9
II) An amino acid sequence which is at least 75% identical to any of the sequences in I).

**15.** An isolated nucleic acid sequence which is complementary to a sequence defined in any of claims 8 to 10.

**16.** An immunogenic composition/vaccine comprising:

I) An isolated virus as defined in any of claims 1 to 7;
II) An isolated or recombinant amino acid sequence/an isolated or recombinant protein as defined in claim 13;
III) An isolated nucleic acid sequence as defined in any of claims 8 to 10; or
IV) A vector as defined in claim 11.

**17.** The immunogenic composition/vaccine according to claim 16 comprising a virus as defined in any of claims 1 to 7, and a pharmaceutically acceptable carrier.

**18.** The immunogenic composition/vaccine according to claim 16 or 17, wherein said virus is either attenuated or inactivated.

**19.** The immunogenic composition/vaccine according to any of claims 16 to 18, wherein said virus is formulated in a water-in-oil emulsion.

**20.** The immunogenic composition/vaccine according to any of claims 16 to 19, wherein said virus or said isolated or recombinant amino acid sequence or protein is combined with other immunologic agents.

**21.** A method of detecting a virus according to any of claims 1 to 7 in a sample, comprising contacting said sample with a nucleic acid sequence as described in any of claims 8-10, or 15; or with an antibody which is raised against the amino acid sequence as set forth in any of SEQ ID NOs: 5-9.

**22.** Diagnostic kit comprising a nucleic acid sequence as described in any of claims 8-10, or 15; or an antibody as described in claim 14.

**23.** A method of manufacturing a vaccine as described in any of claims 16 to 20, said method comprising :

I) Growing a virus as defined in any of claims 1-7 in a cell culture, such as until a cytopathogenic effect is observed
II) Harvesting the virus; and
III) Optionally inactivating the virus.

**24.** A virus as defined in any of claims 1 to 7, an isolated nucleic acid sequence according to any of claims 8-10, or an isolated or recombinant amino acid sequence/an isolated or recombinant protein as defined in claim 13 for use in medicine.

**25.** Use of a virus as defined in any of claims 1 to 7, an isolated nucleic acid sequence according to any of claims 8-10, or an isolated or recombinant amino acid sequence/an isolated or recombinant protein as defined in claim 13 for the manufacture of a immunological composition for preventing infections with salmonid calicivirus or reducing the incidence of such infections, or for treatment of such infections.

**Patentansprüche**

**1.** Isoliertes nicht umhülltes positivsträngiges RNA-Virus, wobei das Virus ausgewählt ist aus der Gruppe, bestehend aus:

I) dem Virusisolat A2-G01, welches unter dem Budapest-Abkommen mit der Europäischen Sammlung von Zellkulturen (ECACC), Health Protection Agency, Porton Down, Salisbury, Wiltshire (UK), SP4 OJG UK am 7. Januar 2010 unter Hinterlegungsnummer 10010701 hinterlegt wurde; und
II) einem Virus, welches in seinem Genom eine Ribonukleinsäuresequenz umfasst, die nach reverser Transkription und Zweitstrangsynthese eine Sequenz bereitstellt, welche wenigstens 65% identisch ist zu der Sequenz, wie in SEQ ID NO:1 gezeigt, und/oder wenigstens 65% identisch ist mit der Sequenz, wie in SEQ ID NO: 2 gezeigt.

**2.** Virus gemäß Anspruch 1, wobei das Genom des Virus einen offenen Leserahmen umfasst, der eine Sequenz codiert, ausgewählt aus der Gruppe, bestehend aus

I) der Aminosäuresequenz, wie in einer der SEQ ID NO:5, SEQ ID NO:6, SEQ ID NO:7, SEQ ID NO:8 und/oder SEQ ID NO:9 gezeigt;
II) einer Aminosäuresequenz, welche wenigstens 75% identisch ist mit einer der Sequenzen in I).

**3.** Virus gemäß Anspruch 1 oder 2, wobei das Genom des Virus eine RNA-abhängige RNA-Polymerase codiert, umfassend eine Aminosäuresequenz, ausgewählt aus der Gruppe, bestehend aus

I) der Aminosäuresequenz, wie in SEQ ID NO:6 gezeigt;
II) einer Aminosäuresequenz, welche wenigstens 75% identisch ist mit einer Sequenz in I).

**4.** Virus gemäß Anspruch 1 oder 2, wobei das Genom des Virus ein Hüllprotein codiert, umfassend eine Aminosäuresequenz, ausgewählt aus der Gruppe, bestehend aus

I) der Aminosäuresequenz wie in SEQ ID NO:8, SEQ ID NO:7 und/oder SEQ ID NO:9 gezeigt;

II) einer Aminosäuresequenz, welche wenigstes 75% identisch ist mit der Sequenz in I).

5. Virus gemäß einem der vorherigen Ansprüche, wobei das Virus ein attenuiertes oder inaktiviertes Virus ist.

6. Virus gemäß einem der vorherigen Ansprüche, wobei das Virus erhältlich ist durch Züchten des Virusisolats A2-G01, welches mit Hinterlegungsnummer 10010701 hinterlegt wurde, in einer Zellkultur, für beispielsweise eine oder mehrere Passagen und/oder bis ein zytopathogener Effekt erreicht wird.

7. Virus gemäß einem der vorherigen Ansprüche, wobei das Virus in der Lage ist, an einen Antikörper zu binden, welcher gegen das Virusisolat A2-G01, welches mit der Hinterlegungsnummer 10010701 hinterlegt wurde, gerichtet ist und/oder in der Lage ist, an einen Antikörper zu binden, welcher gegen die Aminosäuresequenz, wie in einer der SEQ ID NOs:5-9 beschrieben, gerichtet ist, wobei der Antikörper ein polyklonaler Antikörper (Antiserum) oder ein monoklonaler Antikörper ist.

8. Isolierte Nucleinsäuresequenz, welche eine Aminosäuresequenz codiert, die ausgewählt ist aus der Gruppe, bestehend aus

I) der Aminosäuresequenz, wie in einer der SEQ ID NO:5, SEQ ID NO:6, SEQ ID NO:7, SEQ ID NO:8 und/oder SEQ ID NO:9 gezeigt,
II) der Aminosäuresequenz, welche wenigstens 75% identisch ist mit einer der Sequenzen in I).

9. Isolierte Nucleinsäuresequenz gemäß Anspruch 8, wobei die Nucleinsäuresequenz eine Sequenz umfasst oder aus einer Sequenz besteht, ausgewählt aus der Gruppe, bestehend aus

I) der Nucleinsäuresequenz, wie in SEQ ID NO:1 und/oder SEQ ID NO:2 gezeigt;
II) einer Subsequenz der Nucleinsäuresequenz, wie in SEQ ID NO:1 oder SEQ ID NO:2 gezeigt, wobei die Subsequenz eine Länge von 20-7400 konsekutiven Nucleinsäureresten hat; und
III) einer Nucleinsäuresequenz, welche wenigstens 75% identisch mit einer der Sequenzen in I) oder II) ist.

10. Isolierte Nucleinsäuresequenz gemäß Anspruch 9, in welcher die Subsequenz in II) ausgewählt ist aus der Gruppe, bestehend aus:

i) einer Sequenz, umfassend Nucleinsäurereste 1568-2377 der Sequenz, wie in SEQ ID NO:1 gezeigt
ii) einer Sequenz, umfassend Nucleinsäurereste 2842-3355 der Sequenz, wie in SEQ ID NO:1 gezeigt
iii) einer Sequenz, umfassend Nucleinsäurereste 2872-4435 der Sequenz, wie in SEQ ID NO:1 gezeigt
iv) einer Sequenz, umfassend Nucleinsäurereste 3356-4435 der Sequenz, wie in SEQ ID NO:1 gezeigt
v) einer Sequenz, umfassend Nucleinsäurereste 3356-3862 der Sequenz, wie in SEQ ID NO:1 gezeigt; und
vi) einer Sequenz, umfassend Nucleinsäurereste 3637-4435 der Sequenz, wie in SEQ ID NO:1 gezeigt.

11. Vektor, umfassend eine Nucleinsäuresequenz gemäß einem der Ansprüche 8 bis 10.

12. Wirtszelle, umfassend die isolierte Nucleinsäuresequenz gemäß einem der Ansprüche 8 bis 10; und/oder den Vektor gemäß Anspruch 11.

13. Isolierte oder rekombinante Aminosäuresequenz/isoliertes oder rekombinantes Protein, umfassend oder bestehend aus eine(r) oder mehrere(n) Sequenzen, ausgewählt aus der Gruppe, bestehend aus

I) der Aminosäuresequenz, wie in einer der SEQ ID NO:5-9 gezeigt;
II) einer Subsequenz einer der Aminosäuresequenzen wie in einer der SEQ ID NO:5-7 gezeigt, welche eine Länge von 30-200 konsekutiven Aminosäureresten hat, oder einer Subsequenz einer der Aminosäuresequenzen, wie in entweder SEQ ID NO:8 oder SEQ ID NO:9 gezeigt, welche eine Länge von 30-170 konsekutiven Aminosäureresten hat;
III) einer Aminosäuresequenz, welche wenigstens 75% identisch mit den Sequenzen in I) oder II) ist.

14. Antikörper, welcher gerichtet ist gegen eine Aminosäuresequenz, ausgewählt aus der Gruppe, bestehend aus

I) der Aminosäuresequenz, wie in einer der SEQ ID NO:5, SEQ ID NO:6, SEQ ID NO:7, SEQ ID NO:8 und/oder SEQ ID NO:9 gezeigt;

II) einer Aminosäuresequenz, welche wenigstens 75% identisch mit einer der Sequenzen in I) ist.

**15.** Isolierte Nucleinsäuresequenz, welche zu einer Sequenz, wie in einem der Ansprüche 8 bis 10 definiert, komplementär ist.

**16.** Immunogene Zusammensetzung/Impfstoff, umfassend:

I) ein isoliertes Virus, wie in einem der Ansprüche 1 bis 7 definiert;
II) ein(e) isolierte oder rekombinante Aminosäuresequenz/isoliertes oder rekombinantes Protein, wie in Anspruch 13 definiert;
III) eine isolierte Nucleinsäuresequenz, wie in einem der Ansprüche 8 bis 10 definiert; oder
IV) ein Vektor wie in Anspruch 11 definiert.

**17.** Immunogene Zusammensetzung/Impfstoff gemäß Anspruch 16, umfassend ein Virus, wie in einem der Ansprüche 1 bis 7 definiert, und einen pharmazeutisch verträglichen Trägerstoff.

**18.** Immunogene Zusammensetzung/Impfstoff gemäß Anspruch 16 oder 17, wobei das Virus entweder attenuiert oder inaktiviert ist.

**19.** Immunogene Zusammensetzung/Impfstoff gemäß einem der Ansprüche 16 bis 18, wobei das Virus in einer Wasser-in-Öl-Emulsion formuliert ist.

**20.** Immunogene Zusammensetzung/Impfstoff gemäß einem der Ansprüche 16 bis 19, wobei das Virus oder die/das isolierte oder rekombinante Aminosäuresequenz oder Protein mit anderen immunologischen Agenzien kombiniert ist.

**21.** Verfahren zur Detektion eines Virus gemäß einem der Ansprüche 1 bis 7 in einer Probe, umfassend das Inkontaktbringen der Probe mit einer Nucleinsäuresequenz, wie in einem der Ansprüche 8-10 oder 15 beschrieben oder mit einem Antikörper, welcher gegen die Aminosäuresequenz, wie in einer der SEQ ID NOs: 5-9 gezeigt, gerichtet ist.

**22.** Diagnostischer Kit, umfassend eine Nucleinsäuresequenz, wie in einem der Ansprüche 8-10 oder 15 beschrieben oder einen Antikörper, wie in Anspruch 14 beschrieben.

**23.** Verfahren zur Herstellung eines Impfstoffes, wie in einem der Ansprüche 16 bis 20 beschrieben, wobei das Verfahren umfasst:

I) Züchten eines Virus, wie in einem der Ansprüche 1-7 beschrieben, in einer Zellkultur, bis beispielsweise ein zytopathogener Effekt beobachtet wird
II) Ernten des Virus; und
III) optionales Inaktivieren des Virus.

**24.** Virus wie in einem der Ansprüche 1 bis 7 definiert, isoliert Nucleinsäuresequenz gemäß einem der Ansprüche 8-10 oder eine isolierte oder rekombinante Aminosäuresequenz/ein isoliertes oder rekombinantes Protein, wie in Anspruch 13 definiert, zur Verwendung in der Medizin.

**25.** Verwendung eines Virus wie in jedem der Ansprüche 1 bis 7 definiert, einer isolierten Nucleinsäuresequenz gemäß einem der Ansprüche 8-10, oder einer isolierten oder rekombinanten Aminosäuresequenz/eines isolierten oder rekombinanten Proteins, wie in Anspruch 13 definiert, zur Herstellung einer immunologischen Zusammensetzung zur Prävention von Infektionen mit Salmonid Calicivirus oder der Verminderung der Inzidenz solcher Infektionen oder zur Behandlung solcher Infektionen.

## Revendications

**1.** Virus à ARN à brin positif non enveloppé isolé, dans lequel ledit virus est sélectionné dans le groupe constitué par :

I) l'isolat viral A2-G01 déposé conformément au traité de Budapest à l'European Collection of Cell culture (ECACC), Health Protection Agency, Porton Down, Salisbury, Wiltshire (RU), SP4 OJG UK le 7 janvier 2010 sous le numéro de dépôt 10010701 ; et
II) un virus qui comprend dans son génome une séquence d'acide ribonucléique qui par transcription inverse et synthèse de second brin fournit une séquence qui est identique à au moins 65 % à la séquence représentée par SEQ ID NO : 1, et/ou qui est identique à au moins 65 % à la séquence représentée par SEQ ID NO : 2.

**2.** Virus selon la revendication 1, dans lequel le génome dudit virus comprend un cadre ouvert de lecture codant pour une séquence sélectionnée dans le groupe constitué par

I) la séquence d'acides aminés représentée par l'une quelconque parmi SEQ ID NO : 5, SEQ ID NO : 6, SEQ ID NO : 7, SEQ ID NO : 8 et/ou SEQ ID NO : 9
II) une séquence d'acides aminés qui est identique à au moins 75 % à l'une quelconque des séquences de I).

**3.** Virus selon la revendication 1 ou 2, dans lequel le génome dudit virus code pour une ARN polymérase ARN-dépendante comprenant une séquence d'acides aminés sélectionnée dans le groupe constitué par

I) la séquence d'acides aminés représentée par SEQ ID NO : 6 ;
II) une séquence d'acides aminés qui est identique à au moins 75 % à la séquence de I).

**4.** Virus selon la revendication 1 ou 2, dans lequel le génome dudit virus code pour une protéine d'enveloppe comprenant une séquence d'acides aminés sélectionnée dans le groupe constitué par

I) la séquence d'acides aminés représentée par SEQ ID NO : 8, SEQ ID NO : 7 et/ou SEQ ID NO : 9 ;
II) une séquence d'acides aminés qui est identique à au moins 75 % à la séquence de I).

**5.** Virus selon l'une quelconque des revendications précédentes, ledit virus étant un virus atténué ou inactivé.

**6.** Virus selon l'une quelconque des revendications précédentes, dans lequel ledit virus peut être obtenu par la croissance de l'isolat viral A2-G01 déposé sous le numéro de dépôt 10010701 sur une culture de cellules, notamment pendant un ou plusieurs passages et/ou jusqu'à ce qu'un effet cytopathogène soit obtenu.

**7.** Virus selon l'une quelconque des revendications précédentes, ledit virus étant capable de se lier à un anticorps qui est dirigé contre l'isolat viral A2-G01 déposé sous le numéro de dépôt 10010701 et/ou étant capable de se lier à un anticorps qui est dirigé contre la séquence d'acides aminés représentée par l'une quelconque parmi SEQ ID NO : 5 à 9, dans lequel l'anticorps est un anticorps polyclonal (antisérum), ou un anticorps monoclonal.

**8.** Séquence d'acide nucléique isolée codant pour une séquence d'acides aminés sélectionnée dans le groupe constitué par

I) la séquence d'acides aminés représentée par l'une quelconque parmi SEQ ID NO : 5, SEQ ID NO : 6, SEQ ID NO : 7, SEQ ID NO : 8 et/ou SEQ ID NO : 9
II) une séquence d'acides aminés qui est identique à au moins 75 % à l'une quelconque des séquences de I).

**9.** Séquence d'acide nucléique isolée selon la revendication 8, ladite séquence d'acide nucléique comprenant ou consistant en une séquence sélectionnée dans le groupe constitué par :

I) la séquence d'acide nucléique représentée par SEQ ID NO : 1 et/ou SEQ ID NO : 2 ;
II) une sous-séquence de la séquence d'acide nucléique représentée par SEQ ID NO : 1 ou SEQ ID NO : 2, dans laquelle la sous-séquence présente une longueur allant de 20 à 7400 résidus d'acide nucléique consécutifs ; et
III) une séquence d'acide nucléique qui est identique à au moins 75 % à l'une quelconque des séquences de I) ou II).

**10.** Séquence d'acide nucléique isolée selon la revendication 9 dans laquelle ladite sous-séquence de II) est sélectionnée dans le groupe constitué par :

i) une séquence comprenant les résidus d'acide nucléique 1568 à 2377 de la séquence représentée par SEQ ID NO : 1
ii) une séquence comprenant les résidus d'acide nucléique 2842 à 3355 de la séquence représentée par SEQ ID NO : 1
iii) une séquence comprenant les résidus d'acide nucléique 2872 à 4435 de la séquence représentée par SEQ ID NO : 1
iv) une séquence comprenant les résidus d'acide nucléique 3356 à 4435 de la séquence représentée par SEQ ID NO : 1
v) une séquence comprenant les résidus d'acide nucléique 3356 à 3862 de la séquence représentée par SEQ ID NO : 1 et
vi) une séquence comprenant les résidus d'acide nucléique 3637 à 4435 de la séquence représentée par SEQ ID NO : 1.

**11.** Vecteur comprenant une séquence d'acide nucléique selon l'une quelconque des revendications 8 à 10.

**12.** Cellule hôte comprenant la séquence d'acide nucléique isolée selon l'une quelconque des revendications 8 à 10 ; et/ou le vecteur selon la revendication 11.

**13.** Séquence d'acides aminés isolée ou recombinante/protéine isolée ou recombinante comprenant ou consistant en une ou plusieurs séquences sélectionnées dans le groupe constitué par

I) la séquence d'acides aminés représentée par l'une quelconque parmi SEQ ID NO : 5 à 9 ;
II) une sous-séquence de l'une quelconque des séquences d'acides aminés représentées par l'une quelconque parmi SEQ ID NO : 5 à 7, qui présente une longueur de 30 à 200 résidus d'acide aminé consécutifs, ou une sous-séquence de l'une quelconque des séquences d'acides aminés représentées par SEQ ID NO : 8 ou SEQ ID NO : 9, qui présente une longueur de 30 à 170 résidus d'acide aminé consécutifs ;
III) une séquence d'acides aminés qui est identique à au moins 75 % aux séquences de I) ou II).

**14.** Anticorps, qui est dirigé contre une séquence d'acides aminés sélectionnée dans le groupe constitué par

I) la séquence d'acides aminés représentée par l'une quelconque parmi SEQ ID NO : 5, SEQ ID NO : 6, SEQ ID NO : 7, SEQ ID NO : 8 et/ou SEQ ID NO : 9
II) une séquence d'acides aminés qui est identique à au moins 75 % à l'une quelconque des séquences de I).

**15.** Séquence d'acide nucléique isolée qui est complémentaire à une séquence définie dans l'une quelconque des revendications 8 à 10.

**16.** Composition immunogène/vaccin comprenant :

I) un virus isolé tel que défini dans l'une quelconque des revendications 1 à 7 ;
II) une séquence d'acides aminés isolée ou recombinante/une protéine isolée ou recombinante telle que définie dans la revendication 13 ;
III) une séquence d'acide nucléique isolée telle que définie dans l'une quelconque des revendications 8 à 10 ; ou
IV) un vecteur tel que défini dans la revendication 11.

**17.** Composition immunogène/vaccin selon la revendication 16 comprenant un virus tel que défini dans l'une quelconque des revendications 1 à 7, et un support pharmaceutiquement acceptable.

**18.** Composition immunogène/vaccin selon la revendication 16 ou 17, dans lequel ledit virus est atténué ou inactivé.

**19.** Composition immunogène/vaccin selon l'une quelconque des revendications 16 à 18, dans lequel ledit virus est formulé dans une émulsion eau dans huile.

**20.** Composition immunogène/vaccin selon l'une quelconque des revendications 16 à 19, dans lequel ledit virus ou ladite séquence d'acides aminés ou protéine isolée ou recombinante est combiné avec d'autres agents immunologiques.

**21.** Procédé de détection d'un virus selon l'une quelconque des revendications 1 à 7 dans un échantillon, comprenant la mise en contact dudit échantillon avec une séquence d'acide nucléique telle que décrite dans l'une quelconque des revendications 8 à 10, ou 15 ; ou avec un anticorps qui est dirigé contre la séquence d'acides aminés représentée par l'une quelconque parmi SEQ ID NO : 5 à 9.

**22.** Kit de diagnostic comprenant une séquence d'acide nucléique telle que décrite dans l'une quelconque des revendications 8 à 10, ou 15 ; ou un anticorps tel que décrit dans la revendication 14.

**23.** Procédé de préparation d'un vaccin tel que décrit dans l'une quelconque des revendications 16 à 20, ledit procédé comprenant :

I) la croissance d'un virus tel que défini dans l'une quelconque des revendications 1 à 7 dans une culture de cellules, notamment jusqu'à ce qu'un effet cytopathogène soit observé
II) la collecte du virus ; et
III) optionnellement l'inactivation du virus.

**24.** Virus tel que défini dans l'une quelconque des revendications 1 à 7, séquence d'acide nucléique isolée selon l'une quelconque des revendications 8 à 10, ou séquence d'acides aminés isolée ou recombinante/protéine isolée ou recombinante telle que définie dans la revendication 13 pour son utilisation en médecine.

**25.** Utilisation d'un virus tel que défini dans l'une quelconque des revendications 1 à 7, d'une séquence d'acide nucléique isolée selon l'une quelconque des revendications 8 à 10, ou d'une séquence d'acides aminés isolée ou recombinante/d'une protéine isolée ou recombinante telle que définie dans la revendication 13 pour la préparation d'une composition immunologique destinée à prévenir les infections par le calicivirus des salmonidés ou à réduire l'incidence de telles infections, ou à traiter de telles infections.

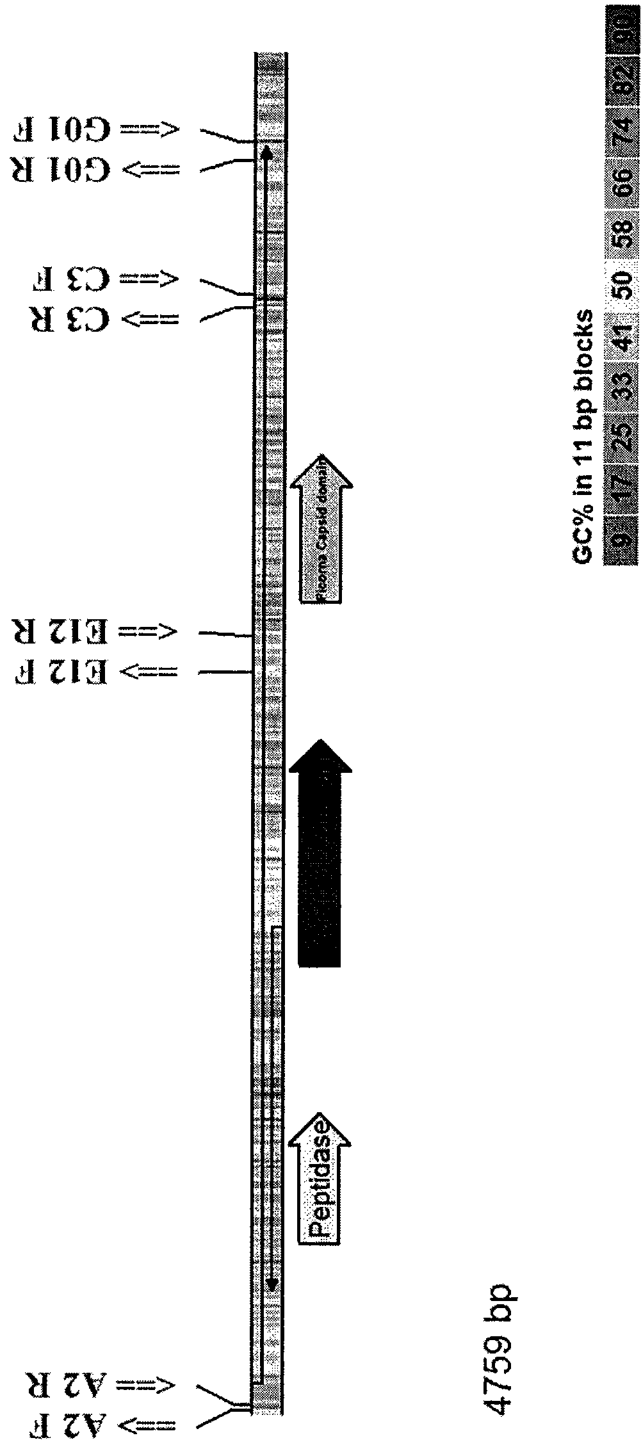
G01 F
G01 R
C3 F
C3 R
E12 R
E12 F
A2 R
A2 F
Peptidase
4759 bp
GC% in 11 bp blocks
9
17
25
33
41
50
58
66
74
82
90

Fig. 1

A.

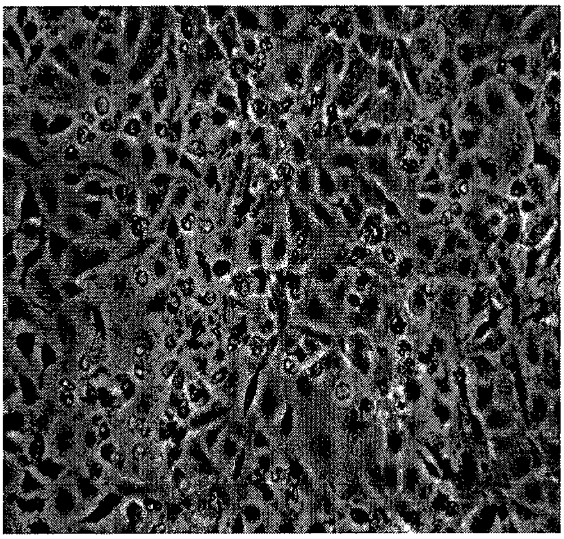

B.

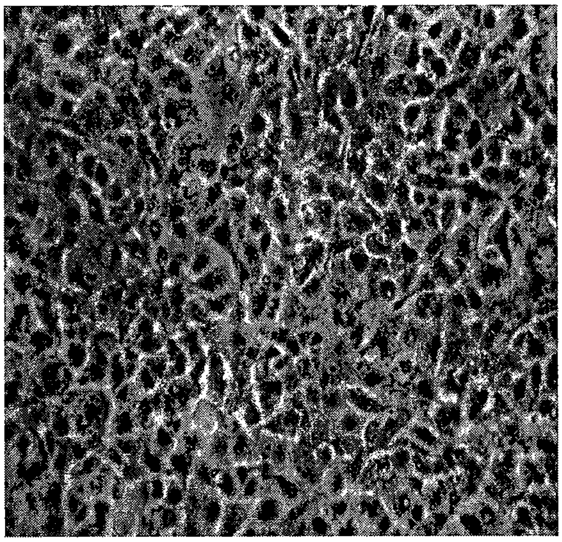

Fig. 2

MSVAQVGAGEDPLKVLLSFERHNVPGLNMLTSFSSVVKQIQIQGKTRTHP 50
KSLVCSINDAKLVLKIPPCSVKFADAVVNFWSYEGKLRCALVKEGVKESA 100
GLGFSTSPSVDPFAFDQVVEDEYELPKSISGALLNVMTTVLGMVAMAMEA 150
KVTFHTPTEEDEKGPARRVTRSYRPVRDSDFQPAGRLWAEDRREMGYTER 200
ITFDSNNWIVPCFDAAGCPKGWAISTPKGLLANRHVIAGTTRLGHQKVGE 250
SVVLNYGDTDLCLIKNTKNPYQLCKAPFSRPIVGEILYQLKPSGMQRARV 300
HSFVRVANPSGKLVEMCLVEGGVSDPGDCGLPWVRRVGRSVELVGLAAGT 350
RGARLMVVPLGVDPAETQWHARTHNYTFLHQTQYFGIVGDDKSMMPSDKV 400
WEGGDEGVMAKHSSEVFFKPGQDNSVSRAAVDASKEYLEHLVPEEKRERW 450
GVLSTVKSLDMCTAAGPSYGTVKQQVFNPDGAPVK**QHAATFWRGVNNPCD** 500
**GKCRISLKDELRPAKKRELGLTRPIFCFDVHDTVRVKSQIGSGLQALADT** 550
**CGTHIWSVGISQQNGTWGQVCERLSKWRYAMDADFGRWDSTNSHPLMKQA** 600
**AEVLASLATRDHREEVEEDLSRMLSAQTQFGPTMTGLPSGLVCTAQMNCT** 650
**SHLLTINDILLGHGAAPVGSMGCPLDFVSYGDDIVLAMDDPKVATWLING** 700
**WKKRGFQATSAAKTGPPQCGKLQDMTFIKRSFKKVDGEWRAPLEAASIWK** 750
**GLSWM**RGHTSYDHSEGVQNGDLTGTRAVGVFQSVMSEFWQHGKEVFEAAK 800
KLIISYARERKLRLPVIIPPYAQFNPKEVLADYNRLAGESSVSMLSVSWH 850
TGGTGEVQPVSQTEGPGGENVGEVVGLPALATGLAHQTAGIETALATSGG 900
PTVQLDPANRERFVMVPGGMVSVRTNMTRGSVVWRKKISPGINMWTNLLS 950
SMYNAWCGGFEVMIVVGANNFIGGKFLVAYFPPNVASGSYSVEQVTAFPH 1000
AILDIRLMDNVLLACSDIKYVLWHPTNAAPEDPIAVGGEVVVYLLTNIVT 1050
AGNSGNVLTLDMSIFSRPMPDFDFNFLMPIS**LGGSGGPNDLDMQAAGRAL** 1100
**CTPATAGGRGWHVIDVLVATQQSSTVAGDMFASTVRMSGTSPYCIPYPAA** 1150
**DGMIVEAIKVSTTDYKLLCYTPDGTPWDVGTDKASKTLPLCWMAYNSASD** 1200
**HTVLTWYKSDGNLEHTNGKITLASVGAQPHYVPYAAAEIPAATRIAVTGS** 1250
**KDGAALTNRESPASPPSFTPNNGESLILYAGLRKTGQKGLTLSTQEMITV** 1300
**FMESPRVTSMCGLFNVSDATGATTVQAKLYPNGVLTTGTAASAIYYTGPI** 1350
**CFTYVGMVPVDYKLNPPAGGTNANVLDLVERLDKWLVLQERSQVGASASS** 1400
**QASLTQQLLSESLGSIKRRPLKSKLETLDSDEESFETILQA** *

Fig. 3

```
Southampto : FTISP------NNTPGDILFDLQLGPHLNPFLSHLSQMYNGWVGNMRVRI : 44
Norwalk_vi : FTISP------NNTPGDVLFDLSLGPHLNPFLLHLSQMYNGWVGNMRVRI : 44
Feline_cal : FSFHTSVNWSTSETQGKILFKQSLGPLLNPYLTHLAKLYVAWSGSVDVRF : 50
Rabbit_hem : EYYNDVFTWSVADAPGSILYTVQHSPQNNPFTAVLSQMYAGWAGGMQFRF : 50
Porcine_en : YTNVT---WTTRQPTGTILARMSLGPGLNPYTLHLSAMWAGWGGSFEIKV : 47
Caliciviru : YTLFGTVSWNANAGPGTILTVGRLGPGMNPYTQHIAAMYGGWAGGMDIRI : 50
Newbury_ag : YTLFGTVSWNANAGPGTILTVGRLGPGMNPYTQHIAAMYGGWAGGMDIRI : 50
Sapporo_vi : ERTFA---WNDRMPTGTFLGSISLHPNINPYTSHLSGMWAGWGGSFEVRL : 47
European_b : EFYNDVFTWSVTDAPGSILYTVQHSPQNNPFTQVLSQMYAGWAGGMQFRF : 50
San_Miguel : ESYHTAVNWSTTEAQGKILFSRALSPELNPYLRHISSLYSTWSGGIDVRF : 50
Human_cali : ETVSP------RNAPGEILWSAPLGPDLNPYLSHLSRMYNGYAGGFEVQV : 44
Murine_nor : ESISP------RNTPGEILFDLALGPGLNPYLAHLSAMYTGWVGNMEVQL : 44
Tulane_vir : ETVSS------ATQPGSVLLELEISPEINLYTSHLFRMYAGWSGGFSLKL : 44
Feline_cal : ESFHTSVNWSTSETQGKILFKQSLGPLLNPYLEHLSKLYVAWSGSVEVRF : 50
PHARMAQ_Ca : VSVRT------NMTRGSVVWRKKISPGINMWTNLLSSMYNAWCGGFEVMI : 44
```

```
Southampto : LLAGNAFSAGKIIVCCVPPGFTSSSLTIAQATLEPHVIADVRTLEPIEMP : 94
Norwalk_vi : MLAGNAFTAGKIIVSCIPPGFGSHNLTIAQATLEPHVIADVRTLDPIEVP : 94
Feline_cal : SISGSGVFGGKLAAIVVPPGIDP--VQSTSMLQYPHVIFDARQVEPVIFS : 98
Rabbit_hem : IVAGSGVFGGRLVRAVIPPGIEIG--PGLEVRQEPHVVIDARSLEPVTIT : 98
Porcine_en : IISGSGMYAGKLLCALIPPGVDPS--AVDQPGAEPHALVDARITDGVTFT : 95
Caliciviru : TIAGSGFIGGTLAVAAIPPGVDPE---SVNVLRMPHVLIDARGGVPLEVT : 97
Newbury_ag : TIAGSGFIGGTLAVAAIPPGVDPE---SVNVLRMPHVLIDARGGVPLEVT : 97
Sapporo_vi : SISGSGVFAGRIIASVIPPGVDPS--SIRDPGVLPHAFVDARITEPVSFM : 95
European_b : IVAGSGIFGGRLVCAIIPPGIQIQ--PGLEVRQEPHVVIDARSLEPVTIT : 98
San_Miguel : TVSGSGVFGGKLAALIVPPGIEPV--ESPTMLQYPHVLFDARQTEPVIFT : 98
Human_cali : ILAGNAFTAGKVIFAAVPPNFPTEGLSPSQVTMEPHIIVDVRQLEPVLIP : 94
Murine_nor : VLAGNAFTAGKVVVALVPPYFPKGSLTTAQITCEPHVMCDVRTLEPIQLP : 94
Tulane_vir : LVAGNAFSAGKLIAAIIPPNIEVPN-SAYLLTGEPHEILDFRTADSMEII : 93
Feline_cal : SISGSGVFGGKLAAIVVPPGVDPI--QSTSMLQYPHVLFDARQVEPVIFT : 98
PHARMAQ_Ca : VVGANNFIGGKFLVAYFPPNVASGSYSVEQVTAEPHAILDIRLMDNVLLA : 94
```

Fig. 4

```
Southampto : LEDVRNVLYHTND----NQPTMRLVCMLYTPIRTGGGSGNSDSFVVAGRV : 140
Norwalk_vi : LEDVRNVLFHNNDR---NQQTMRLVCMLYTPIRTGGGTG--DSFVVAGRV : 139
Feline_cal : IPDLRSTLYHLMS----DTDTTSLVIMVYNDLINPYANDS-NSSGCIVTV : 143
Rabbit_hem : MPDLRPNMYHPTGD---PGLVPTLVLSVYNNLINPFGGS---TSAIQVTV : 142
Porcine_en : LGDVRAVDYHETGV---GGAIASLALYVYQPLINPFETA---VSAAMVTI : 139
Caliciviru : LEDIRTSLYHPMGD----ANTASLVIAVMTGLINPLGTD---TLSVTVQL : 140
Newbury_ag : LEDIRTSLYHPMGD----TNTASLVIAVMTGLINPLGTD---TLSVTVQL : 140
Sapporo_vi : IPDVRAVDYHRMDG---AEPTCSLGFWVYQPLLNPFSTTA--VSTCWVSV : 140
European_b : MPDLRPEMYHPTGN---PGLVPTLVVSVYNNLINPFGGT---TSAIQVTV : 142
San_Miguel : IPDIRKTLYHSMDD----TDTTRLVIMVYNELINPYEQSE-PKSSCSITV : 143
Human_cali : LPDVRNNFYHYNQS---HDSTLKLIAMLYTPIRANNAGDD--VFTVSCRV : 139
Murine_nor : LLDVRRVLWHATQD---QEESMRLVCMLYTPIRTNSPGDE--SFVVSGRL : 139
Tulane_vir : APDIKNIDYHFRGD-----KLGKLVVMVYSPIRSTSADFE-----IEIKL : 133
Feline_cal : IPDLRSTLYHLMSD----TDTTSLVIMVYNDLINPYANDS-NSSGCIVTV : 143
PHARMAQ_Ca : CSDIKYVLWHPTNAAPEDPIAVGGEVVVYLLTNIVTAGNSGNVLTLDMSI : 144
```

Fig. 4, cont.

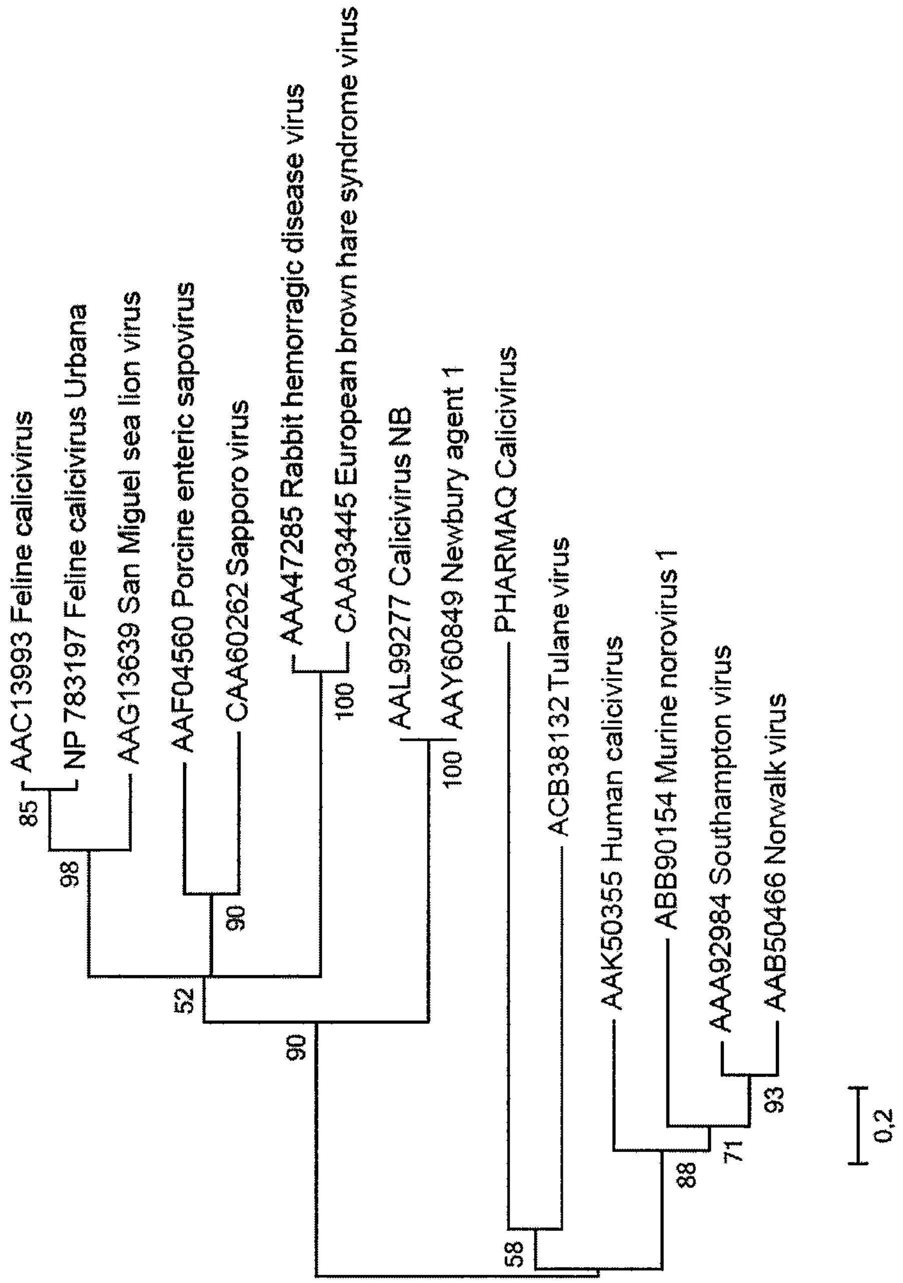
AAC13993 Feline calicivirus
NP 783197 Feline calicivirus Urbana
AAG13639 San Miguel sea lion virus
AAF04560 Porcine enteric sapovirus
CAA60262 Sapporo virus
AAA47285 Rabbit hemorragic disease virus
CAA93445 European brown hare syndrome virus
AAL99277 Calicivirus NB
AAY60849 Newbury agent 1
PHARMAQ Calicivirus
ACB38132 Tulane virus
AAK50355 Human calicivirus
ABB90154 Murine norovirus 1
AAA92984 Southampton virus
AAB50466 Norwalk virus
85
98
90
100
52
100
90
58
88
71
93
0,2

Fig. 5

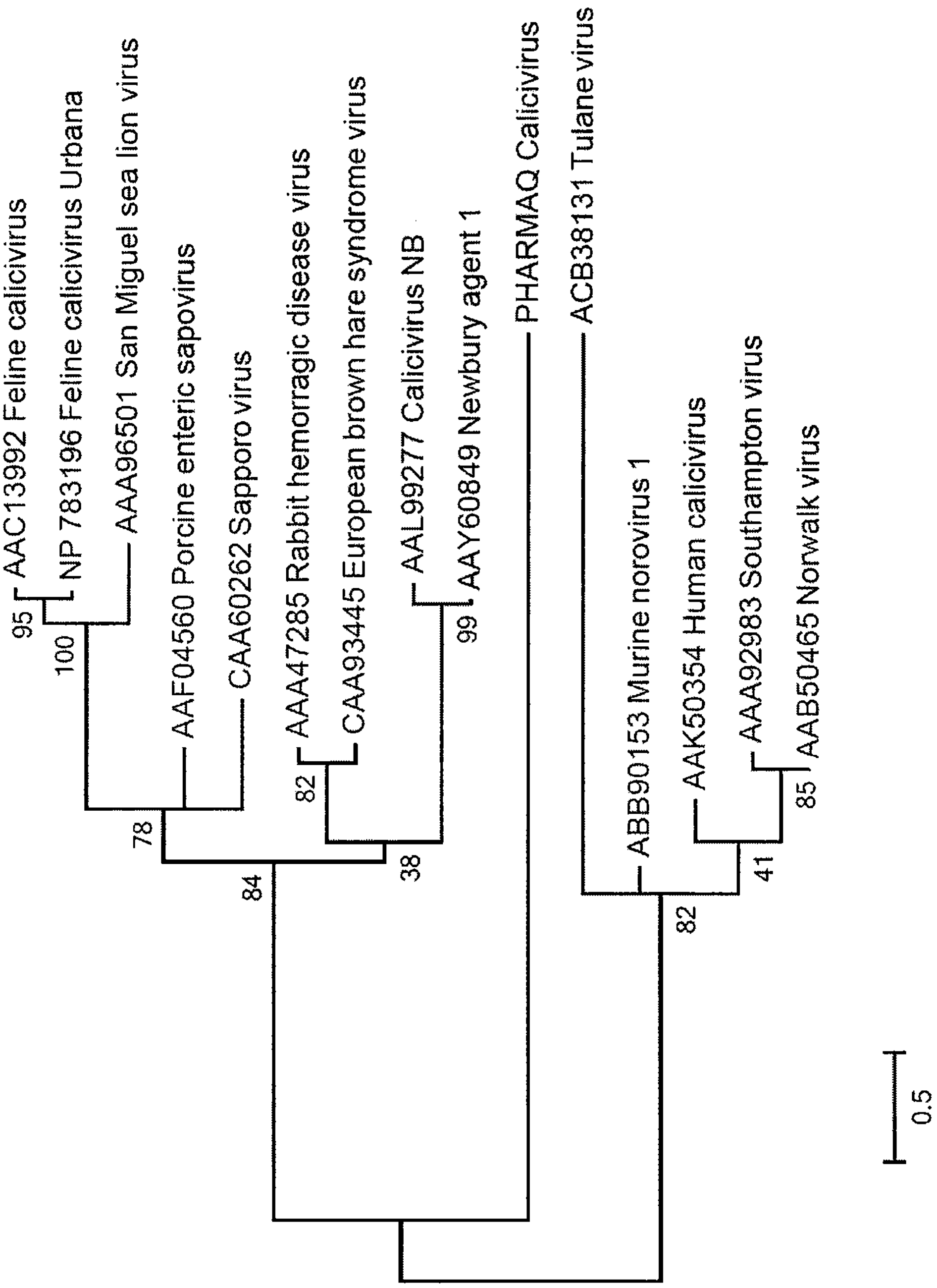

AAC13992 Feline calicivirus
NP 783196 Feline calicivirus Urbana
AAA96501 San Miguel sea lion virus
AAF04560 Porcine enteric sapovirus
CAA60262 Sapporo virus
AAA47285 Rabbit hemorragic disease virus
CAA93445 European brown hare syndrome virus
AAL99277 Calicivirus NB
AAY60849 Newbury agent 1
PHARMAQ Calicivirus
ACB38131 Tulane virus
ABB90153 Murine norovirus 1
AAK50354 Human calicivirus
AAA92983 Southampton virus
AAB50465 Norwalk virus
95
100
78
82
99
38
84
82
41
85
0.5


Fig. 6

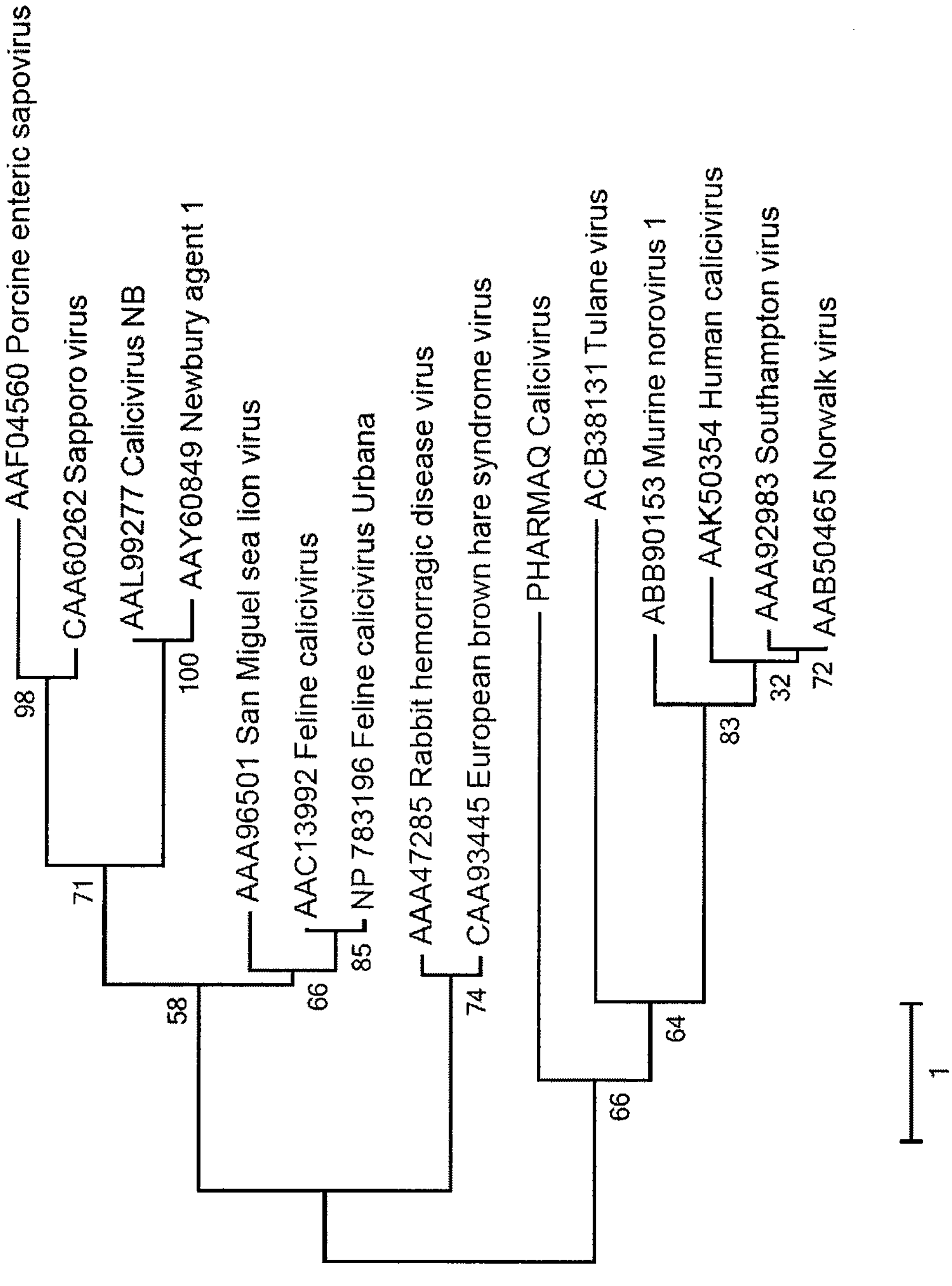
AAF04560 Porcine enteric sapovirus
CAA60262 Sapporo virus
AAL99277 Calicivirus NB
AAY60849 Newbury agent 1
AAA96501 San Miguel sea lion virus
AAC13992 Feline calicivirus
NP 783196 Feline calicivirus Urbana
AAA47285 Rabbit hemorragic disease virus
CAA93445 European brown hare syndrome virus
PHARMAQ Calicivirus
ACB38131 Tulane virus
ABB90153 Murine norovirus 1
AAK50354 Human calicivirus
AAA92983 Southampton virus
AAB50465 Norwalk virus
98
100
71
85
66
58
74
32
72
83
64
66
1

Fig. 7

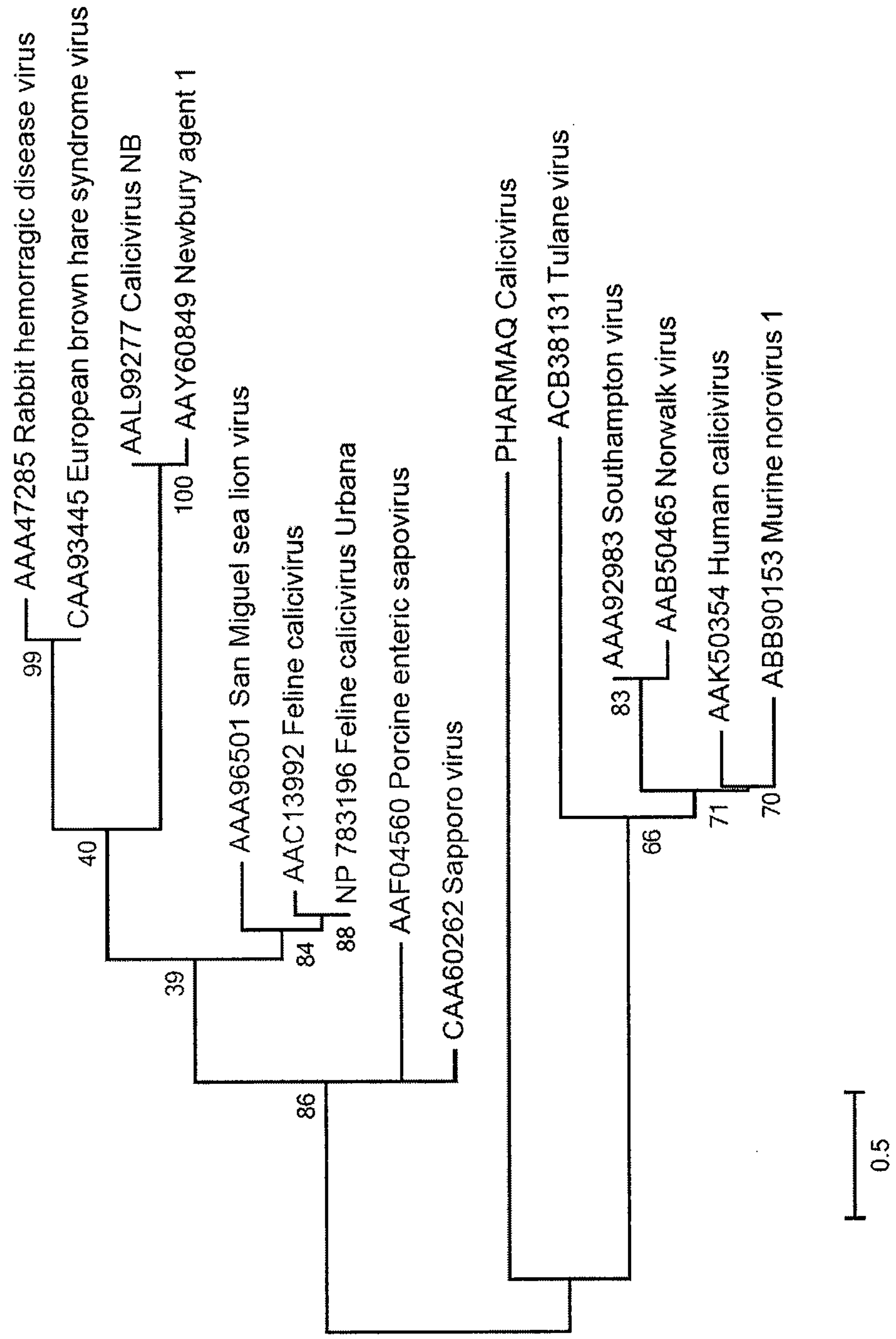
AAA47285 Rabbit hemorragic disease virus
CAA93445 European brown hare syndrome virus
AAL99277 Calicivirus NB
AAY60849 Newbury agent 1
AAA96501 San Miguel sea lion virus
AAC13992 Feline calicivirus
NP 783196 Feline calicivirus Urbana
AAF04560 Porcine enteric sapovirus
CAA60262 Sapporo virus
PHARMAQ Calicivirus
ACB38131 Tulane virus
AAA92983 Southampton virus
AAB50465 Norwalk virus
AAK50354 Human calicivirus
ABB90153 Murine norovirus 1
99
100
40
84
88
39
86
83
71
70
66
0.5

Fig. 8

ECACC

Health Protection Agency

# Health Protection Agency, Porton Down
# and
# European Collection of Cell Cultures

This document certifies that

Virus AQUATIC CALICIVIRUS

Deposit Reference 10010701

has been accepted as a patent deposit, in accordance with The Budapest Treaty of 1977, with the European Collection of Cell Cultures on 07 January 2010

..................................

ECACC Patent Supervisor

Health Protection Agency Culture Collections, Centre for Emergency Preparedness and Response, Porton Down, Salisbury, SP4 0JG, United Kingdom
+44 (0) 1980 612512 +44 (0) 1980 611315 hpacultures@hpa.org.uk www.hpacultures.org.uk

ECACC NCTC NCPV NCPF

Fig. 9

APPENDIX 3

Page 14

BUDAPEST TREATY ON THE INTERNATIONAL RECOGNITION OF THE DEPOSIT OF MICROORGANISMS FOR THE PURPOSES OF PATENT PROCEDURE

INTERNATIONAL FORM

TO
DR PAL NILSEN
PHARMAQ AS
P.O. BOX 267 SKOYEN

OSLO
N-0213
NORWAY

NAME AND ADDRESS OF DEPOSITOR

I. IDENTIFICATION OF THE MICROORGANISM

| Identification reference given by the DEPOSITOR: | Accession number given by the INTERNATIONAL DEPOSITARY AUTHORITY: |
|---|---|
| AQUATIC CALICIVIRUS | 10010701 |

II. SCIENTIFIC DESCRIPTION AND/OR PROPOSED TAXONOMIC DESIGNATION

The microorganism identified under I above was accompanied by:

[ X ] A scientific description

[ ] A proposed taxonomic designation

(Mark with a cross where applicable)

III. RECEIPT AND ACCEPTANCE

This International Depository Authority accepts the microorganism identified under I above, which was received by it on 07 January 2010 (date of the original deposit)[1]

IV. RECEIPT OF REQUEST FOR CONVERSION

The microorganism identified under I above was received by this International Depository Authority on (date of the original deposit) and A request to convert the original deposit to a deposit under the Budapest Treaty was received by it on (date of receipt of request for conversion)

IV. INTERNATIONAL DEPOSITORY AUTHORITY

Name: ECACC Patent Supervisor
Address: European Collection of Cell cultures (ECACC)
Centre for Emergency Preparedness and Response
The Health Protection Agency
Porton Down
Salisbury SP4 0JG

Signature(s) of person(s) having the power to represent the International Depository Authority or of authorized officials(s):

Date: 28 JUN 2010

1 Where Rule 6.4(d) applies, such date is the date on which the status of international depositary authority was acquired

Fig. 9, cont.

APPENDIX 3

Page 24

BUDAPEST TREATY ON THE INTERNATIONAL RECOGNITION OF THE DEPOSIT OF MICROORGANISMS FOR THE PURPOSES OF PATENT PROCEDURE

INTERNATIONAL FORM

TO
DR PAL NILSEN
PHARMAQ AS
P.O. BOX 267 SKOYEN

OSLO
N-0213
NORWAY

NAME AND ADDRESS OF THE PARTY TO WHOM THE VIABILITY OF STATEMENT IS ISSUED

VIABILITY STATEMENT
Issued pursant to Rule 10.2 by the INTERNATIONAL DEPOSITARY AUTHORITY identified on the following page

| I. DEPOSITOR | II. IDENTIFICATION OF THE MICROORGANISM |
|---|---|
| Name: DR PAL NILSEN<br>PHARMAQ AS<br>P.O. BOX 267 SKOYEN<br>Address: OSLO<br>N-0213<br>NORWAY | Accession number given by the INTERNATIONAL DEPOSITORY AUTHORITY:<br>10010701<br>Date of the deposit or of the transfer:<br>07 January 2010 |

II. VIABILITY STATEMENT

The viability of the microorganism identified under II above was tested on 07 January 2010 [2]. On that date, the said microorganism was

[X] [3] viable

[ ] [3] no longer viable

1 Indicate the date of the original deposit or, where a new deposit or a transfer has been made, the most relevant date (date of the new deposit or date of the transfer).

2 In the cases referred to in Rule 10.2 (a) (ii) and (iii), refer to the most recent viability test.

3 Mark with a cross the applicable box.

Form BP/4 (first page)

Fig. 9, cont.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 109942 A
- EP 180564 A
- EP 242380 A
- NO 20101604


### Non-patent literature cited in the description


- **COTHIA et al.** *EMBO j.,* 1986, vol. 5 (4), 823-826
- **JC AGUILAR ; EG RODRIGUEZ.** *Vaccine,* 2007, vol. 25, 3752-3762
- **D. ENDOH.** *Nucleic Acid Research,* 2005, vol. 33 (6
- **PASTORIAN, K.** *Analytical Biochemistry,* 2000, vol. 283, 89-98
- **SMITH AW ; BOYT PM J.** *Zoo Wildlife Med,* 1990, vol. 21, 3-23
- **BERKE et al.** *J Med Virol,* 05 August 1997, vol. 2 (4), 419-424
- **SMITH et al.** *Diseases of Aquatic organisms,* 1986, vol. 2, 73-80
- **ROGNES T.** ParAlign: a parallel sequence alignment algorithm for rapid and sensitive database searches. *Nucleic Acids Research,* 2001, vol. 29, 1647-1652
- **SMITH TF ; WATERMAN MS.** *Journal of Molecular Biology,* 1981, vol. 147, 195-197
- **WATSON JD ; HOPKINS NH ; ROBERTS JW ; STEITZ JA ; WEINER AM.** *Molecular biology of the gene, Chapter 24 The extraordinary diversity of eucaryotic viruses,* 1987, 898-961
- **ELENA, SF ; SANJUÁN, R et al.** *Journal of Virology,* 2005, vol. 79, 11555-11558
- **PASTORIAN, K. et al.** *Analytical Biochemistry,* 2000, vol. 283, 89-98
- **ENDOH, D. et al.** *Nucleic Acid Research,* 2005, vol. 33 (6
- **RAA, J.** *Reviews in Fisheries Science,* 1996, vol. 4 (3), 229-228
- **AGUILAR, JC ; RODRIGUEZ, EG.** *Vaccine,* 2007, vol. 25, 3752-3762